# EUROPEAN PATENT SPECIFICATION

(11) **EP 3 634 224 B1**
(45) Date of publication and mention of the grant of the patent: **09.12.2026**
(21) Application number: 18812652.8
(22) Date of filing: 05.06.2018
(51) Int. Cl.: A61B 5/389, A61B 5/24, A61B 5/296, A61B 5/30, A61N 1/04, A61N 1/05

(54) **TRANSMEMBRANE SENSOR TO EVALUATE NEUROMUSCULAR FUNCTION**
TRANSMEMBRANSENSOR ZUR BEURTEILUNG DER NEUROMUSKULÄREN LFUNKTION
CAPTEUR TRANSMEMBRANAIRE POUR ÉVALUER UNE FONCTION NEUROMUSCULAIRE

(30) Priority: 05.06.2017 US 201762515364 P
(43) Date of publication of application: 15.04.2020
(73) Proprietor: Powell Mansfield, Inc., San Diego, California 92128 (US)
(72) Inventor: MANSFIELD, Perry T., San Diego, California 92128 (US); STUDER, Christopher D., San Diego, California 92128 (US); KOESKE, Timothy J., San Diego, California 92108 (US)
(74) Representative: Potter Clarkson
(86) International application number: PCT/US2018/036151
(87) International publication number: WO 2018/226759

(56) References cited:
- EP-A1- 1 637 076
- US-A- 5 212 476
- US-A- 5 212 476
- US-A1- 2003 060 867
- US-A1- 2012 265 107
- US-A1- 2013 150 749
- US-A1- 2016 150 993
- US-A1- 2016 150 993
- US-B1- 6 361 494
- US-B1- 6 567 990
- FAT-CHUN TONY CHANG ET AL: "A procedure for chronic recording of diaphragmatic electromyographic activity", BRAIN RESEARCH BULLETIN, ELSEVIER SCIENCE LTD, OXFORD, GB, vol. 22, no. 3, 1 March 1989 (1989-03-01), pages 561 - 563, XP023995411, ISSN: 0361-9230, [retrieved on 19890301], DOI: 10.1016/0361-9230(89)90112-3
- WONG: "Where Should the Surface Electromyography Reference Contact Be Located?", 21 June 2005 (2005-06-21), XP055563817
- BREITKREUTZ ET AL.: "Skin Basement Membrane: The Foundation of Epidermal Integrity - BM Functions and Diverse Roles of Bridging Molecules Nitrogen and Perlecan", 10 August 2012 (2012-08-10), XP055563819

## Description

### CROSS-REFERENCE TO RELATED APPLICATIONS

This application claims the benefit of U.S. Provisional Application No. 62/515,364, filed June 5, 2017, and titled "TRANSMEMBRANE SENSOR TO EVALUATE NEUROMUSCULAR FUNCTION,"

### FIELD

Devices, systems, and methods herein relate to electromyography (EMG) that may be used in diagnostic and/or therapeutic applications, including but not limited to electrophysiological study of muscles in the body relating to neuromuscular function and/or disorders.

### BACKGROUND

EMG relates to the study of electrical activity occurring in peripheral nerve and muscle tissue. There are typically two types of techniques for recording EMG signals: intramuscular or needle EMG (NEMG) and surface EMG (SEMG). A needle EMG procedure includes inserting a needle electrode directly into the muscle to be examined. Needle EMG is considered the clinical gold standard for assessing an array of neurophysiologic characteristics of muscle tissue for neuromuscular disease and may provide data related to the muscles and nerves (e.g., motor neurons) that control them. For example, NEMG data may permit characterization of neuromuscular function including spontaneous activity, motor unit action potential (MUAP) recruitment, activation, and morphology. However, NEMG is an invasive procedure that necessarily penetrates tissue and which may cause pain, as well as increase the risk of infection and disease transmission. For example, needle insertion may cause swelling and bleeding, and in some instances, viscus perforation. Some areas of the body may be particularly sensitive to insertion of a needle electrode such as the mouth, pharynx, eyes, ears, gastrointestinal (GI) tract, urinary system, myocardium, and the like.

SEMG is a non-invasive and pain free EMG technique that may be used to assess muscle function by receiving electrical activity of one or more muscles from surface electrodes placed on the skin above the muscles to be examined. Surface EMG signals may be recorded over a prolonged period of time from many sites and motor units, and even when the patient is undergoing physical activity. Surface EMG is considered an acceptable technique for kinesiologic analysis of movement disorders. However, SEMG data may have limited spatial resolution relative to NEMG data due to the large surface area of SEMG sensors. For example, SEMG data may be susceptible to mechanical and electrical artifacts as well as cross-talk between adjacent muscles. Therefore, typical SEMG techniques do not reliably permit characterization of insertional activity, spontaneous activity, motor unit size and shape, and/or interference pattern. The American Academy of Neurology has concluded that SEMG is substantially inferior to NEMG for the evaluation of neuromuscular disorders. Therefore, additional devices, systems, and methods for performing electromyography may be desirable. EP 1 637 076 A1 relates to a skin surface sensor system for detecting and processing EMG signals. US 2016/150993 A1 relates to systems, methods and devices for sensing EMG activity. US 5 212 476 A relates to wireless intraoral controller disposed in oral cavity with electrodes to sense E.M.G. signals produced by contraction of the tongue. FAT-CHUN TONY CHANG ET AL: "A procedure for chronic recording of diaphragmatic electromyographic activity", BRAIN RESEARCH BULLETIN, ELSEVIER SCIENCE LTD, OXFORD, GB, vol. 22, no. 3, 1 March 1989 (1989-03-01), pages 561-563, XP023995411, ISSN: 0361-9230, DOI: 10.1016/0361-9230(89)90112-3 relates to a procedure for chronic recording of diaphragmatic electromyographic activity. WONG ET AL: "Painless electroporation with a new needle-free microelectrode array to enhance transdermal drug delivery", JOURNAL OF CONTROLLED RELEASE, ELSEVIER, AMSTERDAM, NL, vol. 110, no. 3, 21 February 2006 (2006-02-21), pages 557-565, XP005270430, ISSN: 0168-3659, DOI: 10.1016/J.JCONREL.2005.11.003 relates to a skin surface needle-free microelectrode array for transdermal drug delivery. GUBELLINI PET AL: "Deep brain stimulation in neurological diseases and experimental models: From molecule to complex behavior", PROGRESS IN NEUROBIOLOGY, ELSEVIER, AMSTERDAM, NL, vol. 89, no. 1, 1 September 2009 (2009-09-01), pages 79-123, XP026497633, ISSN: 0301-0082, DOI: 10.1016/J.PNEUROBIO.2009.06.003 relates to brain stimulation in neurological diseases and experimental models.

### SUMMARY

Hereinafter, several variations and alternatives, which do not form part of the invention but are merely provided to aid understanding, are described together with the invention, which is defined by the claims. Also described herein are sensor assemblies and methods for non-invasively generating an EMG signal corresponding to muscle tissue where the sensor may be positioned directly on a surface of the muscle tissue including any associated membrane (e.g., mucosal, endothelial, synovial), dermal tissue or connective tissue overlying the muscle tissue. These systems and methods may also be used to permit evaluation of neuromuscular function and/or diagnosis of neuromuscular conditions associated with muscle tissue located within a moist body cavity. Conventional non-invasive EMG devices and techniques such as SEMG record electrical activity of a large surface area corresponding to muscle tissue and may have limited accuracy and utility due to muscle cross-talk (e.g., electrical interference from adjacent muscles) and noise due to moisture between a sensor and tissue (e.g., muscle having a mucosal lining). On the other hand, conventional invasive EMG devices and techniques such as NEMG may cause pain and/or damage to muscle tissue, thereby limiting their use in sensitive tissue systems (e.g., internal organ systems) and adding procedural complexity (e.g., use of general anesthesia).

Generally, the systems and methods described herein may use a sensor to contact an intact tissue surface to receive electrical activity signal data of a specific muscle through any overlying membrane without penetrating or piercing a surface of the tissue. The sensor includes a pair of rounded electrodes configured to directly press against and elastically deform the tissue surface so as to form a temporary indentation while the sensor receives electrical activity data of muscle underlying the surface. The sensor may be configured to provide repeatable signal measurements of an isolated muscle rather than a broader surface area encompassing a group of muscles. Neuromuscular function may be characterized and evaluated using the acquired sensor data.

**In** some variations, a sensor assembly is provided, comprising a sensor including a first electrode, a second electrode, and a sensor housing coupling the first and second electrodes. The first and second electrodes may project from a surface of the sensor housing for a projection length and are spaced apart by a spacing distance. A first ratio of the spacing distance to the projection length may be between about 0.075:1 and about 1.5:1.

**In** some of these variations, the first ratio may be between about 0.15:1 and about 0.75:1. In some variations, a second ratio of a diameter of the first and second electrodes to the spacing distance may be between about 0.2:1 and about 5:1. In some of these variations, the second ratio may be between about 0.4:1 and about 2.5:1. In some variations, a third ratio of a diameter of the first and second electrodes to the projection length may be between about 0.075:1 and about 1.5:1. In some of these variations, the third ratio may be between about 0.15:1 and about 0.75:1.

In some variations, the first and second electrodes may each comprise a rounded distal end. The first and second electrodes may be in parallel. The sensor housing may be configured to electrically isolate the first electrode from the second electrode. The first electrode may be configured as a reference electrode and the second electrode may be configured as an active electrode. The spacing distance may be between about 0.2 mm and about 1.0 mm. The projection length may be between about 0.5 mm and about 3 mm.

In some other variations, a sensor assembly is provided, comprising a sensor including a first electrode, a second electrode electrically isolated from the first electrode, and a sensor housing coupling the first and second electrodes. The first and second electrodes may project in parallel from a surface of the sensor housing. A distance between central longitudinal axes of the first and second electrodes may be between about 0.30 mm and about 2.0 mm.

In some variations, the first and second electrodes may project from the surface of the housing for a projection length between about 0.5 mm and about 3 mm. A diameter of the first and second electrodes may be between about 0.1 mm and about 1.0 mm. The distance may be between about 0.60 mm and about 1.5 mm.

In some variations, the sensor assembly may comprise a probe comprising one or more of the sensors and a handle portion. The probe may comprise a first portion and a second portion detachably attached to the first portion. In some of these variations, the first portion may comprise a paddle shape and a radius of curvature of between about 10 cm and about 20 cm. Adjacent sensors may be spaced apart from each other between about 0.5 cm and about 5 cm. In some of these variations, the probe may comprise one or more dental markers. In some variations, the probe may further comprise a rigid catheter. In other variations, the probe may further comprise a flexible catheter.

In some variations, the assembly may further comprise an amplifier coupled to the probe. The amplifier may comprise a pre-amplifier and/or a main amplifier. A controller may be coupled to the probe and the amplifier. The controller may comprise a processor and a memory. The controller may be configured to receive signal data corresponding to electrical activity of muscle tissue using the one or more sensors. The signal data may be amplified and used to generate electromyography data.

Also described here are methods for using a sensor probe. In general, these methods include the steps of advancing and positioning the probe into a body cavity and sensing activity in the muscle tissue using the sensor. The probe may comprise one or more sensors each comprising a first electrode, a second electrode, and a sensor housing coupling the first and second electrodes. The first and second electrodes may project from a surface of the sensor housing for a projection length and may be spaced apart by a spacing distance. A first ratio of the spacing distance to the projection length may be between about 0.075:1 and about 1.5:1. One or more sensors of the probe may be applied directly on an intact tissue surface so as to elastically deform the tissue surface. Signal data corresponding to electrical activity of tissue may be received using one or more sensors without penetrating or piercing the intact tissue surface.

In some variations, the tissue surface may comprise a membrane overlying the tissue surface. The tissue surface may be maintained in an unbroken state while applying one or more sensors of the probe directly on the tissue surface. The signal data may be processed and used to generate electromyography data.

### BRIEF DESCRIPTION OF THE DRAWINGS

FIGS. 1A-1B are illustrative views of an exemplary variation of a bipolar sensor. FIG. 1A is a perspective view and FIG. 1B is a cross-sectional side view.
FIGS. 2A-2E are illustrative views of an exemplary variation of a sensor assembly. FIGS. 2A and 2C are front perspective views, FIG. 2B is a rear perspective view, and FIG. 2D is a detailed partial cut-away perspective view of the sensor assembly. FIG. 2E is a cross-sectional side view of a sensor depicted in FIGS. 2A-2C.
FIGS. 3A-3C are illustrative views of another variation of a sensor assembly. FIG. 3A is a perspective view, FIG. 3B is a side view, and FIG. 3C is a detailed cross-sectional side view of the bipolar sensor depicted in FIG. 3A.
FIGS. 4A-4B are illustrative perspective views of another variation of a sensor assembly and an endoscope. FIG. 4B is a detailed perspective view of the bipolar sensor depicted in FIG. 4A.
FIG. 5 is a block diagram of another variation of a sensor assembly.
FIG. 6 is an illustrative flowchart of a variation of a method of using a sensor probe.
FIG. 7 is an illustrative frontal surface view of the oropharynx.
FIGS. 8A-8B are illustrative views of a hypopharynx. FIG. 8A is an axial surface view of the hypopharynx and FIG. 8B is an axial view of the hypopharynx musculature.
FIG. 9 is an illustrative sagittal cross-sectional view of the hypopharynx and larynx.
FIG. 10 is an illustrative cross-sectional view of the nasopharynx.
FIG. 11 is an illustrative cross-sectional view of a portion of the upper gastrointestinal tract.
FIG. 12 is an illustrative cross-sectional view of the stomach and duodenum
FIG. 13A is a graph of EMG data of a right palatoglossus muscle using an exemplary variation of a sensor assembly. FIG. 13B is a graph of EMG data of a right palatoglossus muscle using a needle electrode. FIG. 13C is a graph of EMG data of a right first dorsal interosseous muscle using a surface electrode.
FIG. 14A is a graph of motor unit action potential data of a right palatoglossus muscle using an exemplary variation of a sensor assembly. FIG. 14B is a graph of motor unit action potential data of a right palatoglossus muscle using a needle electrode. FIG. 14C is a graph of motor unit action potential data of a right first dorsal interosseous muscle using a surface electrode.

### DETAILED DESCRIPTION

Described herein are sensor devices, systems, and methods for use in non-invasive diagnostic procedures of neuromuscular function of tissue in a body cavity or on a surface of an anatomical structure. In some variations, a sensor assembly may be used for measuring electrical activity of one or more muscles. Generally, a non-invasive transmembrane EMG (TM-EMG) sensor may be used to receive electrical activity signal data corresponding to a specific muscle, with the signal data used to generate EMG data. One or more of the sensors may be incorporated into one or more sensor arrays in a probe. The probe and sensor arrays may be configured to contact muscle tissue in a membranous body cavity (e.g., oropharynx, abdominal cavity, pelvic cavity, joint cavity) or other anatomical structure (e.g., eyes), including anatomical structures accessed intraoperatively.

A sensor assembly as described herein may include one or more pairs of closely spaced, atraumatic electrodes in a bipolar or multipolar configuration. For example, a first electrode may be configured as a reference electrode and a second electrode may be configured as an active electrode. The first and second electrodes may be applied against a surface of muscle tissue (that may include a membrane overlying the muscle) and receive electrical activity signal data corresponding to an electrical potential difference (e.g., voltage) of the portion of muscle between the electrodes. Each electrode may comprise a shape to project or extend into the target muscle tissue. For example, the electrodes may comprise a generally cylindrical shape having a semispherical distal end. The electrodes may be applied against the muscle such that muscle tissue contacts the distal end and/or distal portions of the electrode. However, the shape, length, and spacing of the electrodes are such that the contact is atraumatic and does not damage the muscle (e.g., tear, penetrate the surface). Common noise between the first and second electrodes may be reduced due to the close spacing between the first and second electrode, thereby increasing the SNR of the signal and increasing specificity of the signal data. The atraumatic configuration of the sensor further permits stable and reproducible measurements using the sensor assemblies. Furthermore, the sensor assemblies as described herein may be used in areas of the body that are not typically assessed with NEMG and SEMG. For example, the sensor assemblies as described herein may be used within body cavities and their associated internal organ systems during a surgery or invasive procedure. For example, the sensor assemblies may contact moist muscle tissue having an overlying membrane (e.g., mucosal, endothelial, synovial).

In variations where a controller including a processor and memory are coupled to a TM-EMG sensor, the processor may generate EMG data using the signal data received from the TM-EMG sensor. EMG data generated from the sensor data may correspond to native or spontaneous neuromotor activity and/or a superposition of the evoked action potentials of the active motor units in the measured muscle. The EMG data may have a signal-to-noise ratio (SNR) that permits evaluation of neuromuscular function according to parameters such as insertional activity, spontaneous activity, motor unit size and shape, and interference pattern using sensor data acquired from the devices and systems as described herein.

In some variations, a probe having one or more sensors may be disposed in a housing (e.g., probe) having a size and shape matching a contour of the tissue to be evaluated. Intermediate and proximal portions of the probe may comprise a configuration to aid advancement of the probe to a target muscle. For example, portions of the probe may be flexible or rigid. In some of these variations, a probe may be advanced into a body cavity of interest using a delivery device such as a catheter or endoscope.

### I. Sensor

### A. Electrodes

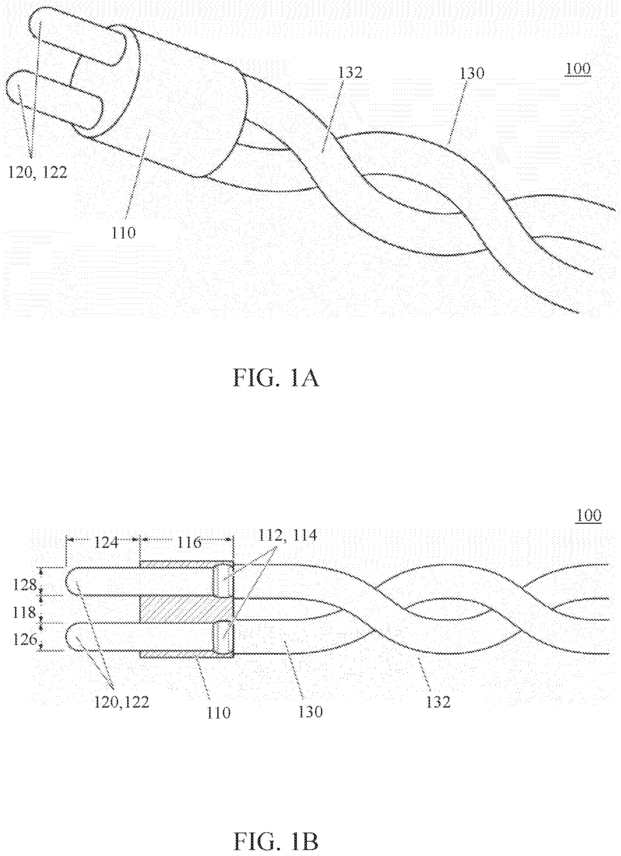

Described herein are electrode sensors for use in measuring electrical activity of one or more muscles. The electrodes may be unipolar, bipolar, or multipolar, and each electrode may comprise a different configuration. FIGS. 1A-1B are illustrative perspective and cross-sectional side views, respectively, of a bipolar sensor (100). The bipolar sensor (100) may comprise a housing (110), a first electrode (120), a second electrode (122), a first lead wire (130), and a second lead wire (132). The housing (110) may have a housing length (116). The housing (110) may couple to the first electrode (120), the second electrode (122), the first lead wire (130), and the second lead wire (132). The first electrode (120) and the second electrode (122) may each project from a surface of the housing (110) for a projection length (124) such that distal portions of the first and second electrodes (120, 122) are uncovered and exposed. The first electrode (120) may have a first diameter (126) and the second electrode (122) may have a second diameter (128). The first and second electrodes (120, 122) may be spaced apart by a spacing distance (118). A first connector (112) may couple the first electrode (120) and the first lead wire (130). A second connector (114) may couple the second electrode (122) and the second lead wire (132). For example, the first and second connectors (112, 114) may be weld points for a solder connection, a pin connector, and the like.

The first electrode (120) and the second electrode (122) may comprise an atraumatic configuration to reduce or prevent damage to tissue damage during contact and/or signal acquisition with the sensor (100). For example, each of the electrodes (120, 122) may comprise a cylindrical body and a semi-spherical or other rounded distal end. In other variations, the electrodes may comprise other shapes (e.g., rectangular body, blunted distal end, rounded edges, flat surfaces, protruding surfaces, smooth surfaces, rough surfaces, grooved surfaces, indented surfaces, mixed surfaces) that are atraumatic to tissue. As another example, one or more of the electrodes may comprise a curved shape (e.g., C-shaped) and/or one or more bends.

In some variations, the first electrode (120) and the second electrode (122) may be parallel to each other. In other variations, the first and second electrodes (120, 122) may be angled non-parallel to each other. For example, the first and second electrodes (120, 122) may form a V-shaped projection relative to each other projecting from the housing (110).

In some variations, as shown in FIGS. 1A-1B, the first and second electrodes (120, 122) may have the same configuration (e.g., dimensions, shape, and orientation). In other variations, the first and second electrodes (120, 122) may have different configurations. For example, the sensor (100) may be configured to have a shape corresponding to a muscle to be measured such that one electrode may be longer than the other electrode, and have different diameters and/or shapes. A spacing distance (118) between the electrodes (120, 122) may be based on the submucosal, subendothelial, subsynovial, muscular anatomy.

The electrodes (120, 122) of the sensor (100) may comprise dimensions such that the electrode pair is atraumatic when in contact with muscle tissue. The dimensions described herein permit the electrodes to measure electrical activity of muscle tissue. In some variations, the electrodes (120, 122) may comprise a diameter (126, 128) between about 0.1 mm and about 1.0 mm. In some variations, the electrodes (120, 122) may comprise a diameter (126, 128) between about 0.3 mm and about 0.75 mm. In some variations, the electrodes (120, 122) may comprise a projection length (124) between about 0.5 mm and about 3.0 mm. In some variations, the electrodes (120, 122) may comprise a projection length (124) between about 0.5 mm and about 2.5 mm. In some variations, the electrodes (120, 122) may comprise a projection length (124) between about 1.0 mm and about 2.0 mm. In some variations, the electrodes (120, 122) may comprise a total length (e.g., projection length and insulated length) of between about 0.5 mm and about 5.0 mm.

The dimensions described herein permit the electrodes to measure electrical activity of muscle tissue atraumatically and with specificity to evaluate neuromuscular function of desired tissue. The electrodes (120, 122) of the sensor (100) may comprise a spacing distance (118) configured such that desired muscle tissue may be isolated while permitting a potential difference of muscle between the electrodes to be measured. For example, the electrode spacing (118) of the bipolar sensor (100) disclosed herein is such that common noise between the electrodes (120, 122) may be reduced to thereby improve an SNR of the bipolar sensor signal data. For example, a smaller spacing distance (118) corresponds to a more focused and precise measurement of muscle while a larger spacing distance (118) corresponds to a more general measurement of the muscle. In some variations, the electrodes (120, 122) may comprise a spacing distance (118) between about 0.2 mm and about 1.0 mm. In some variations, the electrodes (120, 122) may comprise a spacing distance (118) between about 0.3 mm and about 0.75 mm. In other variations, the electrodes (120, 122) may comprise a spacing distance between a first central longitudinal axis (e.g., through the center or midpoint) of the first electrode and a second central longitudinal axis of the second electrode may be between about 0.3 mm and about 2.0 mm. In some other variations, the electrodes (120, 122) may comprise a spacing distance between a first central longitudinal axis of the first electrode and a second central longitudinal axis of the second electrode of between about 0.6 mm and about 1.5 mm.

The sensors described herein may permit the electrodes to measure electrical activity of muscle tissue atraumatically and with specificity to evaluate neuromuscular function based on one or more relationship(s) between the dimensions of the electrodes. For example, electrode dimensions including spacing distance, electrode length, and electrode diameter may be related such that the electrodes are spaced close enough to permit voltage measurement of desired muscle tissue and the shape and dimensions of the electrodes are atraumatic to reduce damage to tissue (e.g., tissue piercing). In some variations, a first ratio of the spacing distance (118) to the projection length (124) may be between about 0.075 and about 1.5:1. In some variations, a first ratio of the spacing distance (118) to the projection length (124) may be between about 0.15:1 and about 0.75:1. In some variations, a second ratio of a diameter of the first and second electrodes (120, 122) to the spacing distance (118) may be between about 0.2:1 and about 5:1. In some variations, the second ratio of a diameter of the first and second electrodes (120, 122) to the spacing distance (118) may be between about 0.4:1 and about 2.5:1. In some variations, a third ratio of a diameter of the first and second electrodes (120, 122) to the projection length (124) may be between about 0.075:1 and about 1.5:1. In some variations, the third ratio of a diameter of the first and second electrodes (120, 122) to the projection length (124) may be between about 0.15:1 and about 0.75:1.

The electrodes as described herein may be formed of any biocompatible conductive metal and/or alloy including, but not limited to tungsten, silver, platinum, platinum-iridium, nickel titanium alloys, copper-zinc-aluminum-nickel alloys, copper-aluminum-nickel alloys, combinations thereof, and the like. The lead wires described herein may comprise an electrically conductive wire configured to connect the electrodes of a bipolar sensor to other components of a sensor assembly, such as an amplifier, controller, and the like. The amplifier may comprise a pre-amplifier, either alone or in combination with another amplifier. In some variations, each electrode may be coupled to a respective insulated lead wire. The lead wires (130, 132) of a pair of electrodes (120, 122) may be configured as a twisted pair (e.g., braided). This twisting may reduce the electromagnetic interference and/or crosstalk from other pairs of lead wires in the device. The number of twists per inch may be in the range of about 0.5 to about 5 twists per inch, and different pairs may have different twists per inch. The lead wires as described herein may comprise any length necessary to couple its corresponding electrode to the sensor assembly. In some variations, the lead wire may comprise a length of between about 0.1 m and about 2.0 m. In some variations, the lead wire may comprise a length of between about 0.5 m and about 1.5 m. In some variations, the lead wire may have about the same diameter as its corresponding electrode. The lead wires as described herein may be formed of any electrically conductive metal and/or biocompatible conductive metal and/or alloy including, but not limited to copper, silver, platinum, platinum-iridium, combinations thereof, and the like. In some variations, the lead wires may comprise a touch proof, single pole connector (e.g., DIN 42-802) at a proximal end. In some variations, the lead wires may be stranded or solid.

One or more portions of the lead wires may be flexible or semi-flexible, one or more portions may be rigid or semi-rigid, and/or one or more portions of the lead wires may transition between flexible and rigid configurations. The lead wires described herein may be made of any material or combination of materials. For example, the lead wires may be insulated using one or more polymers (e.g., silicone, polyvinyl chloride, latex, polyurethane, polyethylene, PTFE, nylon).

In some variations, the sensors described herein may comprise a ground electrode and a corresponding ground wire configured to reduce noise. The ground electrode and ground wire may be separate from or integrated with the sensor in a housing. The ground electrode and ground wire may be formed of any biocompatible conductive metal and/or alloy including, but not limited to tungsten, silver, platinum, platinum-iridium, nickel titanium alloys, copper-zinc-aluminum-nickel alloys, copper-aluminum-nickel alloys, combinations thereof, and the like.

In some variations, the sensor may be configured as a multipolar sensor with three or more of the electrodes as described herein. The electrodes of a multipolar sensor may be configured to optimize surface area contact with predetermined muscle tissue, thereby increasing the SNR of the signal and specificity of the signal data.

In some variations, the sensor electrodes, ground electrode and lead wires as described herein may be integrated into a single cable. For example, the cable may comprise one or more layers of shielding and insulation. In some variations, the shielding and insulation layers may be disposed individually over one or more of the sensor and ground electrodes and/or disposed over the cable as a whole. The ground electrode of a single cable may comprise an interwoven mesh or spiral shape with helical, wrapped strands. In some variations, the cable may comprise one or more ground electrodes. For example, the cable may comprise a ground electrode for each sensor electrode. The lead wires of the cable may be stranded or solid. For example, the number of strands may be between about 7 and about 100.

### B. Housing

As shown in FIGS. 1A-1B, the bipolar sensor (100) may comprise a housing (110) configured to physically support and/or protect the electrodes (120, 122), lead wires (130, 132), and connectors (112, 114) coupled therebetween. The housing (110) may be further configured to electrically isolate the first electrode (120) from the second electrode (122). The housing (110) may have any atraumatic configuration that does not damage muscle tissue. The housing (110) may be configured to have any length to support and/or protect the electrodes (120, 122), connectors (112, 114), and lead wires (130, 132), and may be based on the muscle to be evaluated. In some variations, the housing (110) may comprise a length of between about 1.0 mm and about 2.0 mm. In some variations, the housing (110) may comprise a diameter to surround the pair of spaced-apart electrodes (120, 122). The housing as described herein may be formed of any biocompatible non-conductive material including, but not limited to epoxy, Teflon, PVS, ABS plastic, silicone, polyvinyl chloride, latex, polyurethane, polyethylene, PTFE, nylon, combinations thereof, and the like.

### II. Sensor assembly

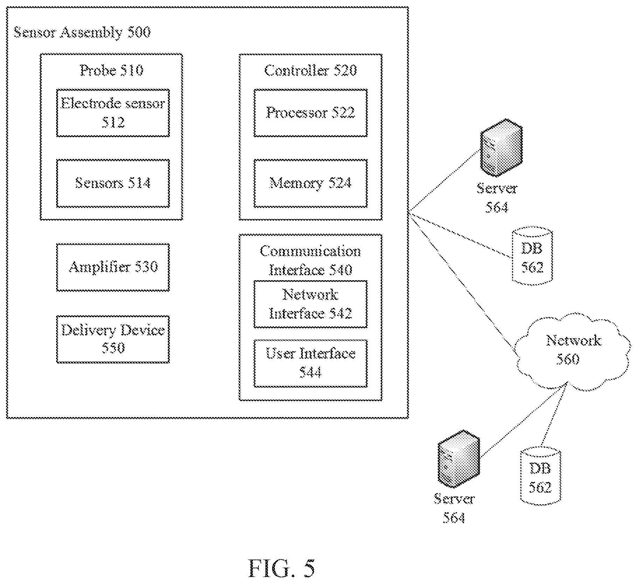

A sensor assembly may include one or more of the components necessary to measure and evaluate muscle tissue using the bipolar or multipolar sensors as described herein. The sensor assembly may couple to one or more computer systems and/or networks. FIG. 5 is a block diagram of another variation of a sensor assembly (500). The sensor assembly (500) may comprise a probe (510) that may be advanced into a body cavity or surface of an anatomical structure and placed against muscle to be evaluated. In some variations, the probe (510) may comprise one or more electrode sensors (512) and/or additional sensors (514). In some variations, the additional sensors (514) may comprise one or more of a thermal sensor, optical sensor (e.g., CCD), light source, proximity sensor, and the like. For example, an optical sensor may permit visualization of a body cavity or anatomical surface that may aid probe placement. The probe (510) may be coupled to a controller (520) configured to receive and process the sensor data from the probe (510). The controller (520) may comprise a processor (522) and a memory (524). In some variations, the sensor assembly (500) may further comprise one or more of an amplifier (530), a communication interface (540), and a delivery device (550). The probe (510) and controller (520) may be coupled to the amplifier (530) that is configured to process the electrode sensor signal data to, for example, increase the SNR of the signal data. The controller (520) may be coupled to the communication interface (540) to permit an operator to control the sensor assembly (500), probe (510), signal processing, data output, etc. The communication interface (540) may comprise a network interface (542) configured to connect the sensor assembly (500) to another system (e.g., Internet, remote server, database) over a wired and/or wireless network. The communication interface (540) may further comprise a user interface (544) configured to permit an operator to directly control the sensor assembly (500). In some variations, the probe (510) may be advanced into a body cavity using a delivery device (550) such as a catheter or endoscope.

### A. Controller

A sensor assembly (500), as depicted in FIG. 5, may comprise a controller (520) in communication with one or more probes (510). The controller (520) may comprise one or more processors (522) and one or more machine-readable memories (524) in communication with the one or more processors (522). The processor (522) may incorporate data received from memory (524) and operator input to control the sensor assembly (500) (e.g., one or more probes (510) and/or delivery devices (550)). The memory (524) may further store instructions to cause the processor (522) to execute modules, processes, and/or functions associated with the sensor assembly (500). The controller (520) may be connected to the one or more probes (510) by wired or wireless communication channels. In some variations, the controller (520) may be coupled to a patient platform or disposed on a medical cart adjacent to the patient and/or operator. The controller (520) may be configured to control one or more components of the sensor assembly (500), such as probe (510), communication interface (540), delivery device (550), and the like.

The controller (520) may be implemented consistent with numerous general purpose or special purpose computing systems or configurations. Various exemplary computing systems, environments, and/or configurations that may be suitable for use with the systems and devices disclosed herein may include, but are not limited to software or other components within or embodied on personal computing devices, network appliances, servers or server computing devices such as routing/connectivity components, portable (e.g., hand-held) or laptop devices, multiprocessor systems, microprocessor-based systems, and distributed computing networks. Examples of portable computing devices include smartphones, personal digital assistants (PDAs), cell phones, tablet PCs, phablets (personal computing devices that are larger than a smartphone, but smaller than a tablet), wearable computers taking the form of smartwatches, portable music devices, and the like, and portable or wearable augmented reality devices that interface with an operator's environment through sensors and may use head-mounted displays for visualization, eye gaze tracking, and user input.

### i. Processor

The processor (522) may be any suitable processing device configured to run and/or execute a set of instructions or code and may include one or more data processors, image processors, graphics processing units, physics processing units, digital signal processors, and/or central processing units. The processor (522) may be, for example, a general purpose processor, Field Programmable Gate Array (FPGA), an Application Specific Integrated Circuit (ASIC), and/or the like. The processor (522) may be configured to run and/or execute application processes and/or other modules, processes and/or functions associated with the system and/or a network associated therewith. The underlying device technologies may be provided in a variety of component types, e.g., metal-oxide semiconductor field-effect transistor (MOSFET) technologies like complementary metal-oxide semiconductor (CMOS), bipolar technologies like emitter-coupled logic (ECL), polymer technologies (e.g., silicon-conjugated polymer and metal-conjugated polymer-metal structures), mixed analog and digital, and/or the like.

### ii. Memory

In some variations, the memory (524) may include a database (not shown) and may be, for example, a random access memory (RAM), a memory buffer, a hard drive, an erasable programmable read-only memory (EPROM), an electrically erasable read-only memory (EEPROM), a read-only memory (ROM), Flash memory, and the like. As used herein, database refers to a data storage resource. The memory (524) may store instructions to cause the processor (522) to execute modules, processes and/or functions associated with the sensor assembly (500), such as probe control, signal data processing, EMG data processing, sensor control, communication, and/or user settings. In some variations, storage may be network-based and accessible for one or more authorized users. Network-based storage may be referred to as remote data storage or cloud data storage. EMG signal data stored in cloud data storage (e.g., database) may be accessible to respective users via a network, such as the Internet. In some variations, database (120) may be a cloud-based FPGA.

Some variations described herein relate to a computer storage product with a non-transitory computer-readable medium (also may be referred to as a non-transitory processor-readable medium) having instructions or computer code thereon for performing various computer-implemented operations. The computer-readable medium (or processor-readable medium) is non-transitory in the sense that it does not include transitory propagating signals per se (e.g., a propagating electromagnetic wave carrying information on a transmission medium such as space or a cable). The media and computer code (also may be referred to as code or algorithm) may be those designed and constructed for the specific purpose or purposes. Examples of non-transitory computer-readable media include, but are not limited to, magnetic storage media such as: hard disks; floppy disks; magnetic tape; optical storage media such as Compact Disc/Digital Video Discs (CD/DVDs); Compact Disc-Read Only Memories (CD-ROMs); holographic devices; magneto-optical storage media such as optical disks; solid state storage devices such as a solid state drive (SSD) and a solid state hybrid drive (SSHD); carrier wave signal processing modules; and hardware devices that are specially configured to store and execute program code, such as Application-Specific Integrated Circuits (ASICs), Programmable Logic Devices (PLDs), Read-Only Memory (ROM), and Random-Access Memory (RAM) devices. Other variations described herein relate to a computer program product, which may include, for example, the instructions and/or computer code disclosed herein.

The systems, devices, and/or methods described herein may be performed by software (executed on hardware), hardware, or a combination thereof. Hardware modules may include, for example, a general-purpose processor (or microprocessor or microcontroller), a field programmable gate array (FPGA), and/or an application specific integrated circuit (ASIC). Software modules (executed on hardware) may be expressed in a variety of software languages (e.g., computer code), including C, C++, Java^{®}, Python, Ruby, Visual Basic^{®}, and/or other object-oriented, procedural, or other programming language and development tools. Examples of computer code include, but are not limited to, micro-code or micro-instructions, machine instructions, such as produced by a compiler, code used to produce a web service, and files containing higher-level instructions that are executed by a computer using an interpreter. Additional examples of computer code include, but are not limited to, control signals, encrypted code, and compressed code.

### B. Amplifier

A sensor assembly (500), as depicted in FIG. 5, may comprise an amplifier (530) coupled to one or more of the probe (510), controller (520), and communication interface (540). The amplifier (530) may be configured to process electrical activity signal data from one or more of the bipolar or multipolar sensors (512) and/or sensors (514). For example, the amplifier (530) may be configured to process the bipolar sensor signal data to improve signal-to-noise (SNR) by reducing artifacts, cross-talk, and increasing spatial resolution. In some variations, the amplifier (530) may comprise one or more of a pre-amplifier, main amplifier and a multi-stage differential amplifier. For example, a differential amplifier (530) may be configured to amplify a voltage difference measured between a pair of electrodes of a multipolar electrode sensor (512). The amplifier (530) may comprise several stages to increase the gain of the SNR ratio by amplification of the voltage signal near the source, prior to the emergence of noise that develops in circuits of the sensor assembly (500). For example, a differential amplifier may reduce artifacts due to AC power and action potentials of distant muscles. In some variations, electrode sensitivity may be set at about 50u V/division, but in other embodiments may be between about 40 uV/division and about 60 uV/division, or between about 30 uV/division and about 100 uV/division, or between about 10 uV/division and about 200 uV/division. The sweep speeds may be set at about 10 ms/division, or may be between about 5 uV/division and about 20 ms/division, or between about 3 uV/division and about 30 ms/division. In some variations, threshold capture may be established at about 100 uV, but in other examples may be between about 50 uV and about 150 uV, or between about 80 uV and about 200 uV.

### C. Communication interface

The communication interface (544) may permit an operator to interact with and/or control the sensor assembly (500) directly and/or remotely. For example, a user interface (544) of the sensor assembly (500) may include an input device for an operator to input commands and an output device for an operator and/or other observers to receive output (e.g., view patient data on a display device) related to operation of the sensor assembly (500). In some variations, a network interface (542) may permit the sensor assembly (500) to communicate with one or more of a network (560) (e.g., Internet), remote server (564), and database (562) as described in more detail herein.

### i. User interface

User interface (544) may serve as a communication interface between an operator and the sensor assembly (500). In some variations, the user interface (544) may comprise an input device and output device (e.g., touch screen and display) and be configured to receive input data and output data from one or more of the probe (510), delivery device (550), input device, output device, network (560), database (562), and server (564). For example, images generated by an optical sensor of a delivery device (550) (e.g., an endoscope) may be processed by processor (522) and memory (524), and displayed by the output device (e.g., monitor display). Sensor data from one or more sensors (512, 514) may be received by user interface (544) and output visually and/or audibly through one or more output devices. As another example, operator control of an input device (e.g., joystick, keyboard, touch screen) may be received by user interface (544) and then processed by processor (522) and memory (524) for user interface (544) to output a control signal to one or more probes (510) and delivery devices (550).

### 1. Output device

An output device of a user interface (544) may output sensor data corresponding to a patient and/or sensor assembly (500), and may comprise one or more of a display device and audio device. The output device may be coupled to a patient platform and/or disposed on a medical cart adjacent to the patient and/or operator. In other variations, the output device may be mounted to any suitable object, such as furniture (e.g., a bed rail), a wall, a ceiling, and may be self-standing.

The display device may be configured to display a graphical user interface (GUI). A display device may permit an operator to view signal data, EMG data, and/or other data processed by the controller (520) such as images of one or more body cavities and tissue. For example, an endoscope comprising an optical sensor (e.g., camera) located in a body cavity or lumen of a patient may be configured to image an internal view of the body cavity and/or muscle tissue to be measured. In some variations, an output device may comprise a display device including at least one of a light emitting diode (LED), liquid crystal display (LCD), electroluminescent display (ELD), plasma display panel (PDP), thin film transistor (TFT), organic light emitting diodes (OLED), electronic paper/e-ink display, laser display, and/or holographic display.

An audio device may audibly output patient data, sensor data, system data, alarms, and/or warnings. For example, the audio device may output an audible warning when monitored patient data (e.g., temperature, heart rate) falls outside a predetermined range or when a malfunction in the probe (510) is detected. In some variations, an audio device may comprise at least one of a speaker, piezoelectric audio device, magnetostrictive speaker, and/or digital speaker. In some variations, an operator may communicate with other users using the audio device and a communication channel. For example, the operator may form an audio communication channel (e.g., VoIP call) with a remote operator and/or observer.

### 2. Input device

Some variations of an input device may comprise at least one switch configured to generate a control signal. The input device may be coupled to a patient platform and/or disposed on a medical cart adjacent to the patient and/or operator. However, the input device may be mounted to any suitable object, such as furniture (e.g., a bed rail), a wall, a ceiling, or may be self-standing. In some variations, the input device may comprise a wired and/or wireless transmitter configured to transmit a control signal to a wired and/or wireless receiver of a controller (520). For example, an input device may comprise a touch surface for an operator to provide input (e.g., finger contact to the touch surface) corresponding to a control signal. An input device comprising a touch surface may be configured to detect contact and movement on the touch surface using any of a plurality of touch sensitivity technologies including capacitive, resistive, infrared, optical imaging, dispersive signal, acoustic pulse recognition, and surface acoustic wave technologies. In variations of an input device comprising at least one switch, a switch may comprise, for example, at least one of a button (e.g., hard key, soft key), touch surface, keyboard, analog stick (e.g., joystick), directional pad, pointing device (e.g., mouse), trackball, jog dial, step switch, rocker switch, pointer device (e.g., stylus), motion sensor, image sensor, and microphone. A motion sensor may receive operator movement data from an optical sensor and classify an operator gesture as a control signal. A microphone may receive audio and recognize an operator voice as a control signal.

### ii. Network interface

As depicted in FIG. 5, a sensor assembly (500) described herein may communicate with one or more networks (560) and computer systems (564) through a network interface (542). In some variations, the sensor assembly (500) may be in communication with other devices via one or more wired and/or wireless networks. The network interface (110) may facilitate communication with other devices over one or more external ports (e.g., Universal Serial Bus (USB), multi-pin connector) configured to couple directly to other devices or indirectly over a network (e.g., the Internet, wireless LAN).

In some variations, the network interface (542) may comprise a radiofrequency receiver, transmitter, and/or optical (e.g., infrared) receiver and transmitter configured to communicate with one or more devices and/or networks. The network interface (542) may communicate by wires and/or wirelessly with one or more of the probe (510), delivery device (550), user interface (544), network (560), database (562), and server (564).

In some variations, the network interface (542) may comprise radiofrequency (RF) circuitry (e.g., RF transceiver) including one or more of a receiver, transmitter, and/or optical (e.g., infrared) receiver and transmitter configured to communicate with one or more devices and/or networks. RF circuitry may receive and transmit RF signals (e.g., electromagnetic signals). The RF circuitry converts electrical signals to/from electromagnetic signals and communicates with communications networks and other communications devices via the electromagnetic signals. The RF circuitry may include one or more of an antenna system, an RF transceiver, one or more amplifiers, a tuner, one or more oscillators, a digital signal processor, a CODEC chipset, a subscriber identity module (SIM) card, memory, and the like. A wireless network may refer to any type of digital network that is not connected by cables of any kind. Examples of wireless communication in a wireless network include, but are not limited to cellular, radio, satellite, and microwave communication. The wireless communication may use any of a plurality of communications standards, protocols and technologies, including but not limited to Global System for Mobile Communications (GSM), Enhanced Data GSM Environment (EDGE), high-speed downlink packet access (HSDPA), wideband code division multiple access (W-CDMA), code division multiple access (CDMA), time division multiple access (TDMA), Bluetooth, near-field communication (NFC), radio-frequency identification (RFID), Wireless Fidelity (Wi-Fi) (e.g., IEEE 802.11a, IEEE 802.11b, IEEE 802.11g and/or IEEE 802.11n), voice over Internet Protocol (VoIP), Wi-MAX, a protocol for email (e.g., Internet Message Access Protocol (IMAP) and/or Post Office Protocol (POP)), instant messaging (e.g., eXtensible Messaging and Presence Protocol (XMPP), Session Initiation Protocol for Instant Messaging and Presence Leveraging Extensions (SIMPLE), and/or Instant Messaging and Presence Service (IMPS)), and/or Short Message Service (SMS), or any other suitable communication protocol. Some wireless network deployments combine networks from multiple cellular networks or use a mix of cellular, Wi-Fi, and satellite communication. In some variations, a wireless network may connect to a wired network in order to interface with the Internet, other carrier voice and data networks, business networks, and personal networks. A wired network is typically carried over copper twisted pair, coaxial cable, and/or fiber optic cables. There are many different types of wired networks including, but not limited to, wide area networks (WAN), metropolitan area networks (MAN), local area networks (LAN), Internet area networks (IAN), campus area networks (CAN), global area networks (GAN), like the Internet, and virtual private networks (VPN). As used herein, network refers to any combination of wireless, wired, public, and private data networks that are typically interconnected through the Internet, to provide a unified networking and information access system.

In some variations, the sensor assembly (500) may comprise an analog-to-digital converter (not shown) configured to convert an analog voltage signal into a digital voltage signal. The accuracy of the signal conversion may depend on the sampling frequency and the number of steps (e.g., vertical resolution). For example, a high sampling frequency and a large number of steps may produce a more accurate digital replica of the received analog signal.

### D. Probe

A probe (510) may comprise one or more of the bipolar or multipolar electrode sensors (512) configured to measure electrical activity of a set of muscles. The probe (510) may comprise a housing configured with a size, shape, and sensor arrangement suited for advancement into a body cavity or surface of an anatomical structure and the muscle(s) to be evaluated. For example, a transoral probe comprising a curved, rigid probe housing including at least two sensor arrays may be configured to contact and measure electrical activity of a group of muscles in an upper airway cavity. In another example, a rigid probe may comprise a single bipolar electrode sensor coupled to a rigid shaft. The probe configurations as described herein are merely illustrative.

In some variations, the devices, systems, and methods may comprise one or more elements described in International Application Serial No. PCT/US2015/018196, filed on February 27, 2015, and titled "SYSTEMS, METHODS AND DEVICES FOR SENSING EMG ACTIVITY," and/or U.S. Provisional Application Serial No. 61/946,259, filed on February 28, 2014, and titled "SYSTEMS, METHODS AND DEVICES FOR SLEEP APNEA,"

### i. Transoral probe

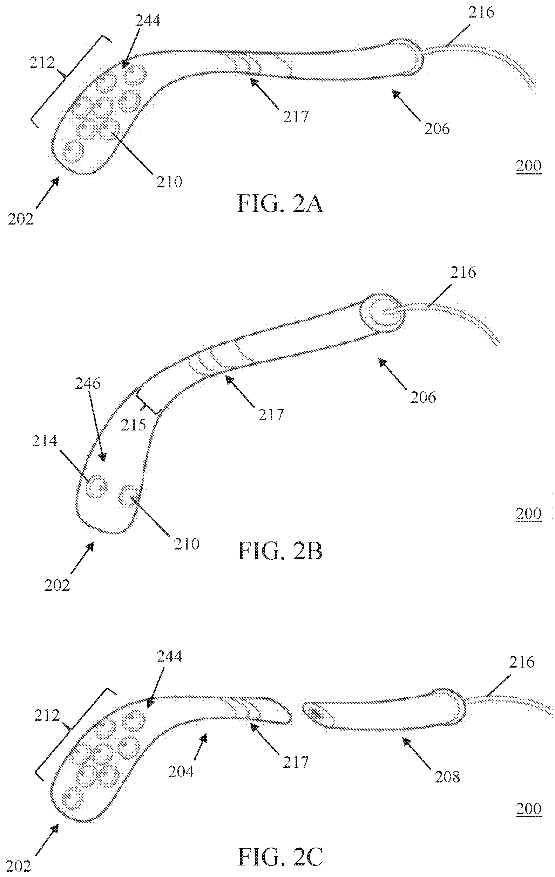

FIGS. 2A-2E are illustrative views of a transoral probe (200). The probe (200) as depicted in the front perspective view of FIG. 2A may comprise a distal portion (202) coupled to a proximal portion (206). A cable (216) (e.g., insulated cable) may extend from the proximal portion (206). The transoral probe (200) as disclosed herein are usable with any of the assemblies (500), sensors (e.g., bipolar sensor (100), multipolar sensor), and methods described herein. For example, each sensor (210) of the probe (200) may comprise a pair of closely spaced, atraumatic electrodes. As another example, cable (216) may couple to a sensor assembly (not shown) including a controller (520) as described herein. The probe (200) may comprise a plurality of bipolar or multipolar sensors (210) arranged in one or more sensor arrays (212, 214). For example, the distal portion (202) of the probe (200) may comprise a first array (212) of bipolar sensors (210) on a first side (e.g., top, front side) of the probe (200). As shown in the rear perspective view of FIG. 2B, a second side (e.g., bottom, rear side) of the probe (200) may comprise a second array (214) of bipolar sensors (210). As illustrated, the first array (212) may comprise eight bipolar sensors (210) and the second array (214) may comprise two bipolar sensors (210). In this example, the first array (212) comprises four sensors (210) arranged along the midline of the distal superior surface of the probe (200), but in other examples there may be 1, 2, 3, 5, 6, 7, 8, 9, 10 or more midline sensors. The midline sensors are flanked by two sensors (210) to each side of the midline, but in other embodiments, 1, 3, 4, 5 or more sensors may be configured to each side of the midline, and the arrangement need not be symmetrical, and the spacing between adjacent sensors may or may not be the same. The flanking sensors (210) are spaced between the most distal and most proximal midline sensors (210), but in other examples, the flanking sensors (210) may be located along the same width location as the most distal or most proximal midline sensors (210). In this example, the second array (214) of sensors (210) located on the inferior surface (246) of the probe (200) comprises one sensor (210) to each side of the midline and spaced from the distal end of the probe (200) by a distance of at least the diameter of the sensor (210), or a distance closer. In other examples, however, other sensor configurations may be used, including the configuration as provided on the superior surface of the probe, including a different number of sensors, sensors with a midline location, or sensors closer or farther from the distal end of the probe.

In some variations, the distal portion (202) and proximal portion (206) may comprise a combined length of between about 5 cm and about 30 cm. In some variations, the distal portion (202) and proximal portion (206) may comprise a combined length of between about 10 cm and about 20 cm. In some variations, the distal portion (202) and proximal portion (206) may comprise a combined length of about 16.5 cm. In some variations, the proximal portion (206) may comprise a length of between about 2 cm and about 20 cm. In some variations, the proximal portion (206) may comprise a length of between about 5 cm and about 10 cm. In some variations, the proximal portion (206) may comprise a length of about 8 cm. In some variations, the distal portion (202) may comprise a width of between about 1.5 cm and about 7 cm. In some variations, the distal portion (202) may comprise a width of between about 3 cm and about 4 cm. In some variations, the distal portion (202) may comprise a width of about 3.5 cm. In some variations, the proximal portion (206) may comprise a circumference of between about 1 cm and about 5 cm. In some variations, the proximal portion (206) may comprise a circumference of between about 2 cm and about 4 cm. In some variations, the proximal portion (206) may comprise a circumference of about 3 cm. In some variations, the distal portion (202) may comprise a paddle shape comprising a length of between about 4 cm and about 10 cm. In some variations, the paddle shape may comprise a length of between about 6 cm and about 8 cm. In some variations, the paddle shape may comprise a length of about 7.3 cm. In some variations, the distal portion (202) may comprise a width (215) of between about 0.5 cm and about 3.5 cm. In some variations, the distal portion (202) may comprise a width (215) of about 1.8 cm. In some variations, the distal portion (202) may comprise a radius of curvature of between about 10 cm and about 20 cm. In some variations, the distal portion (202) may comprise a radius of curvature of about 15 cm. The superior surface (244) of the probe (200) may comprise a convex curvature, and the inferior surface (246) may comprise a concave curvature.

The transoral probe (200) may be configured for placement adjacent to at least one of the soft palate, pharyngeal wall, and tongue of a patient, as described in more detail herein. For example, the size, shape, and other physical characteristics of a probe housing may be configured for an upper airway cavity of a patient to permit evaluation of muscle tissue. In some variations, as shown in FIG. 2C, the probe (200) may comprise a first portion (e.g., distal portion (202)) and a second portion (e.g., proximal portion) detachably attached to the first portion. This may allow, for example, the first portion to be used as a single-use, disposable sensor portion while the second portion may be a sterilizable, reusable portion. A proximal end of the first portion and the distal end of the second portion may each comprise a connector connected to lead wires such as a single pole connector (e.g., DIN 42-802). The first and second portions may remain attached until forced apart by an operator. Lead wires may extend from the bipolar sensors (210) through each of the first and second portions.

In some variations, the probe (200) may comprise one or more dental markers (217). For example, the dental markers (217) may comprise one or more indentations (e.g., notches) and/or protrusions (e.g., grooves) configured for one or more upper or lower teeth to bite into. In some variations, the dental markers (217) may be spaced apart between about 0.5 cm and about 1.5 cm. In some variations, the dental markers (217) may be spaced apart by about 1.0 cm. In some variations, the dental markers (217) may be disposed at least 5 mm away from a proximal end of the distal portion (202).

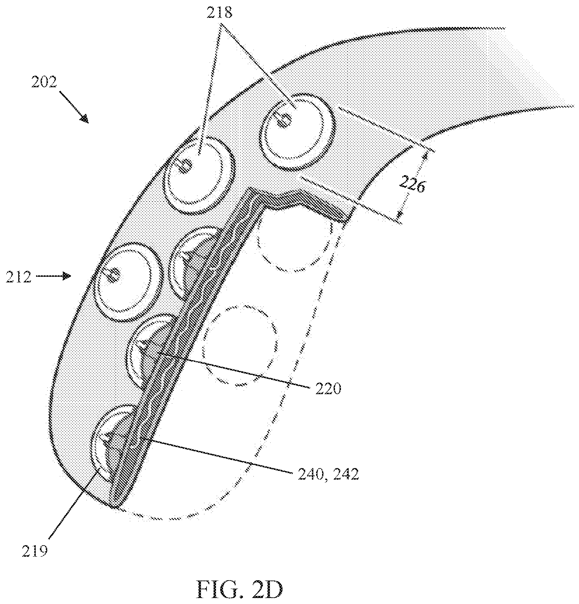

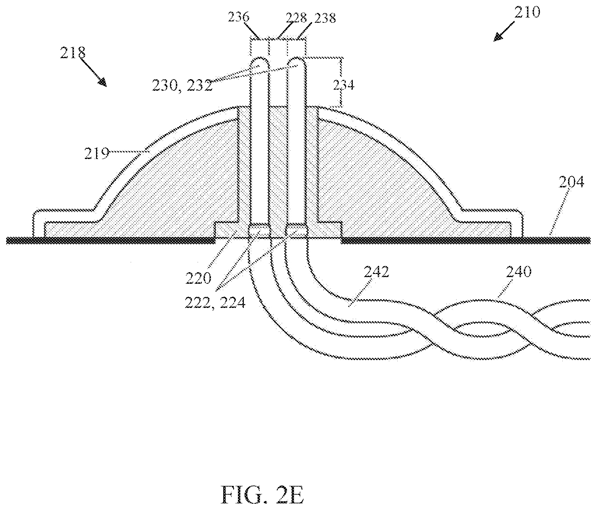

FIG. 2D is a detailed partial cut-away perspective view of a distal portion (202) of the probe (200). FIG. 2E is a cross-sectional side view of one of the bipolar sensors (210) depicted in FIGS. 2A-2D. Each of the bipolar sensors (210) comprises a pair of electrodes within a sensor housing (218). The sensor housing (218) may comprise a housing portion (219) and a housing cavity (220) where a bipolar sensor (210) may be disposed, as shown in FIGS. 2D and 2E. The housing portion (219) may comprise a generally rounded shape. The housing portion (219) may comprise an exterior surface and an inner insulated portion. Each of the two electrodes (230, 232) of a bipolar sensor (210) may be coupled to respective lead wires (240, 242) using respective connectors (222, 224). The sensor housing (218) may comprise a diameter (226) of between about 0.25 cm and about 2 cm. In some variations, the sensor housing (218) may comprise a diameter (226) of about 1.5 cm. In some variations, the bipolar sensors (210) may be spaced apart from each other by at least about 0.5 cm. In some variations, the bipolar sensors (210) may be spaced apart from each other by about 1.5 cm. In some variations, the sensor housings (218) may be spaced apart from each other by at least about 0.1 mm. In some variations, the sensor housings (218) may be spaced apart from each other by about 0.5 cm.

In some variations, a flexible printed circuit may be disposed in the distal portion (202) of the probe (200) and coupled to each of the bipolar or multipolar sensors (210). One or more flexible printed circuits may be configured to electrically connect the bipolar or multipolar sensors (210) to the cable (216). The flexible printed circuits are not particularly limited and may be a single-sided flex circuit, double-sided flex circuit, double access flex circuit, and the like.

The bipolar sensors (210) may comprise a first electrode (230) having a first diameter (236) and a second electrode (232) having a second diameter (238). In some variations, the electrodes (230, 232) may have a spacing distance (228) between each other and a projection length (234) from a surface of the housing portion (219). The shape, dimensions, and materials of the sensor (210) may be the same as those described herein with respect to bipolar sensor (100). The housing portion (219) may be formed on a surface of the first portion (204) (e.g., assembly housing).

### ii. Rigid probe

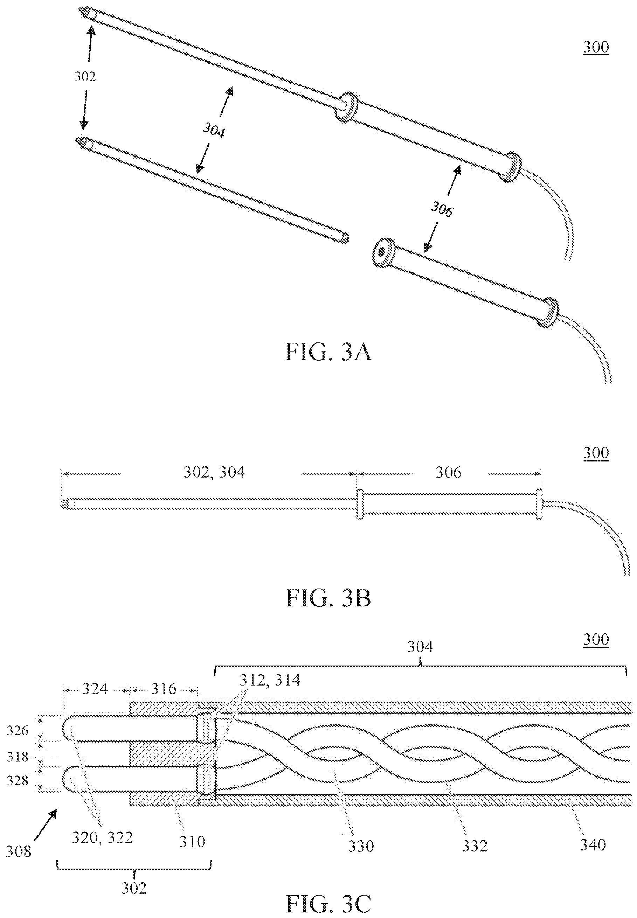

FIGS. 3A-3C are illustrative views of a rigid sensor assembly (300). The sensor assembly (300) may comprise a distal portion (302) (e.g., bipolar or multipolar sensors), intermediate portion (304) (e.g., shaft (304)), and a proximal portion (306) (e.g., handle). The proximal portion (306) may be configured as a handle for an operator to grasp and control the sensor assembly (300). The handle may have any suitable length and shape. The intermediate portion (304) and the proximal portion (306) may each comprise a hollow lumen and house therein one or more of an insulated cable (e.g., lead wires), a power cable, a flexible printed circuit, other electronics, and the like. In some variations, the intermediate portion (304) may comprise a rigid shaft, a semi-flexible shaft, and/or comprise portions having combinations thereof. The intermediate portion (304) described herein may be any elongate body suitable for advancement through at least a portion of one or more body lumens and/or cavities. The intermediate portion (304) may be hollow, partially hollow, and/or partially solid. One or more portions of the intermediate portion (304) may be flexible or semi-flexible, one or more portions may be rigid or semi-rigid, and/or one or more portions may be configured to transition between flexible and rigid configurations. Flexible portions of the intermediate portion (304) may allow it to be navigated through tortuous body lumens to reach a desired target site. The intermediate portion (304) described here may be made of any material or combination of materials. For example, the intermediate portion (304) may comprise one or more metals or metal alloys such as nickel titanium alloys, copper-zinc-aluminum-nickel alloys, copper-aluminum-nickel alloys, combinations thereof, and the like, and/or one or more polymers such as silicone, polyvinyl chloride, latex, polyurethane, polyethylene, PTFE, nylon, combinations thereof, and the like.

The intermediate portion (304) may have any suitable dimensions. For example, the intermediate portion (304) may have any suitable length that allows the assembly (300) to be advanced from a point external to the body to a target location. In some variations, the length of the intermediate portion (304) may be between about 1 cm and about 100 cm. The intermediate portion (304) may have any suitable diameter, such as, for example, about 5.7 French, about 6.1 French, about 7 French, about 8.3 French, between about 5 French and about 9 French, between about 5 French and about 7 French, between about 6 French and about 9 French, and the like. The intermediate portion (304) may be removably attached from the proximal portion (306). In some variations, the distal portion (302) may be removably attached to the intermediate portion (304). In other variations, the distal portion (302) may be fixed to the intermediate portion (304).

The distal portion (302) may comprise one or more bipolar or multipolar sensors (308) each comprising a first electrode (320) having a first diameter (326) and a second electrode (322) having a second diameter (328). In some variations, the electrodes (320, 322) may have a spacing distance (318) between each other and a projection length (324) from a surface of the sensor housing portion (310). The housing portion (310) may have a housing length (316). The shape, dimensions, and materials of the bipolar sensor (308) may be the same as those described herein with respect to any of the bipolar sensors disclosed such as bipolar sensors (100, 200, 430, 512). The first and second electrodes (320, 322) may be coupled to respective lead wires (330, 332) through corresponding connectors (312, 314) (e.g., weld points) as described herein.

In some variations, a flexible printed circuit may be disposed in one or more of the intermediate portion (304) and the distal portion (306) of the assembly (300) and coupled to the bipolar or multipolar sensor (308). One or more flexible printed circuits may be configured to electrically connect the bipolar sensor (308) to a controller. Also, in some examples, the probe may be a flexible or malleable probe, or comprise a combination of one or more rigid sections, flexible sections and malleable sections.

### E. Probe and delivery device

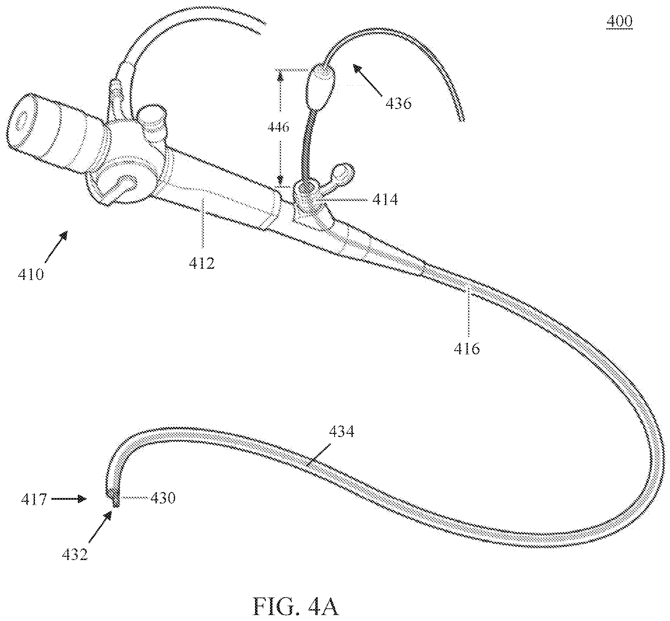

FIGS. 4A-4B are illustrative perspective views of another variation of a sensor system (400) comprising a sensor assembly (430) (e.g., probe) and a delivery device (410) (e.g., access device, visualization device) and configured for one or more of advancement, placement, and visualization of the sensor assembly (430). In some variations, the sensor assembly (430) may be configured to slidably advance through an endoscope (410) to be located topically on membranes in targeted areas of the gastrointestinal (GI) tract to measure EMG activity of muscles associated with GI function such as pharyngeal muscles, a cricopharyngeus, esophageal muscles, a lower esophageal sphincter, a pancreatic sphincter and a bile duct sphincter. In some variations, the sensor assembly may be configured to slidably advance through a catheter of a delivery device to be located topically on membranes in targeted areas of the heart to measure EMG activity of the myocardium. In some variations, the sensor assembly may be configured to pass through an endoscope to be located topically on membranes in targeted urologic areas of the body to measure EMG activity of muscles associated with urologic functions such as a detrusor muscle, a urethral sphincter, a bladder, and the like. In some variations, the delivery device (410) may comprise one or more adult and pediatric flexible endoscopes, laryngoscope, rhino laryngoscope, laryngeal strobe scope, bronchoscope, Zenker's scope, esophagoscope, colonoscope, sigmoidoscope, cystoscope, fiberscope, camera, external light source, imaging sensor, combinations thereof, and the like. The delivery device (430) may be configured to visualize one or more of the sensor probe and anatomic surfaces of a body cavity such as a nasopharynx, oropharynx, hypopharynx, larynx, and esophagus.

In some variations, the delivery device may comprise an optical sensor (e.g., a charged coupled device (CCD) or complementary metal-oxide semiconductor (CMOS) optical sensor) and may be configured to generate an image signal that is transmitted to a display. For example, in some variations, the delivery device may comprise a camera with an image sensor (e.g., a CMOS or CCD array with or without a color filter array and associated processing circuitry). An external light source (e.g., laser, LED, lamp, or the like) may generate light that may be carried by fiber optic cables or one or more LEDs may be configured to provide illumination. For example, a fiberscope comprising a bundle of flexible optical fibers may be configured to receive and propagate light from an external light source. The fiberscope may comprise an image sensor configured to receive reflected light reflected from a body cavity. The image sensor may detect the reflected light and convert it into image signals that may be processed and transmitted for display. The endoscope may have any suitable configuration, for example, it may be a chip-on-the-tip camera endoscope, a three camera endoscope, and the like. The sensor probes may be configured for a forward, side or retro-facing direction.

In some variations, the delivery device (410) may be a catheter for angiography including, transvascular analysis of muscle activity such a myocardium, chordae, cardiac valves including tricuspid, mitral, pulmonic, and aortic valves. In some variations, the sensor assembly may be incorporated into robotic surgical systems, computer navigated surgery systems, and/or minimally invasive surgery systems. This may include using a tool or end effector provided on a robotic arm, catheter, endoscope or minimally invasive diagnostic or surgical device to be positioned on membranes in targeted areas of the gastrointestinal (GI) tract, urinary tract, and oropharyngeal cavity, for example.

The endoscope (410) may comprise a body portion (412) including a port (414) coupled to a catheter (416) having a distal end (417). The sensor assembly (430) may comprise a proximal portion (436) (e.g., handle), a flexible intermediate portion (434), and a distal portion (432). The sensor assembly (430) may be advanced through the port (414) and through the catheter (416) such that the distal portion (432) of the sensor assembly (430) is slidably advanced out of the distal end (417) of the catheter (416). The intermediate portion (434) may be sized to slidably advance within a lumen of the catheter (416). The proximal portion (436) may comprise a handle, insulated lead wires, and the like. In some variations, the proximal portion (436) may comprise a length in the range of between about 1 cm and about 3 m. The proximal portion (436) may be separated from the port (414) by a predetermined distance (446). In some variations, the distance from the port (414) to a handle may be in the range of between about 10 cm and about 30 cm.

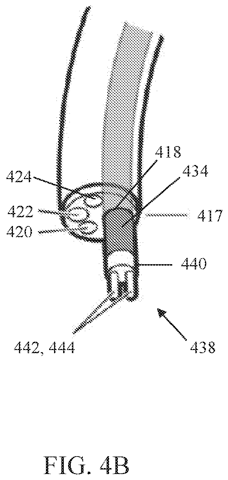

FIG. 4B is a perspective view of the distal end (417) of the catheter (416) and a bipolar sensor (438) of the sensor assembly (430). The distal end (417) of the catheter (416) may comprise a first lumen (418), second lumen (420), third lumen (422), and fourth lumen (424). In some variations, the first lumen (418) may be configured as a sensor assembly lumen for a sensor assembly (430) to slidably advance through. The second lumen (420) may be configured as an optical sensor lumen for an optical sensor (not shown) to be disposed in. A third lumen (422) may be configured as a light source lumen for a light source (not shown) to be disposed in. A fourth lumen (424) may be configured as a guidewire lumen for a guidewire (not shown) to slidably advance through. The number of lumens in the catheter (430) is not particularly limited and may include one, two, three, four, and five or more lumens.

The catheter (416) and intermediate portion (434) described herein may comprise any elongate body suitable for advancement through at least a portion of body lumen. The catheter (416) and intermediate portion (434) may be hollow, partially hollow, and/or partially solid. One or more portions of the catheter (416) and intermediate portion (434) may be flexible or semi-flexible, one or more portions may be rigid or semi-rigid, and/or one or more portions of the catheter (416) and intermediate portion (434) may be changed between flexible and rigid configurations. Flexible portions of the catheter (416) and intermediate portion (434) may allow the catheter (416) and intermediate portion (434) to be navigated (e.g., steerable) through tortuous body lumens to reach a desired target site. The catheter (416) and intermediate portion (434) described here may be made of any material or combination of materials. For example, the catheter (416) and intermediate portion (434) may comprise one or more metals or metal alloys (e.g., e.g., nickel titanium alloys, copper-zinc-aluminum-nickel alloys, copper-aluminum-nickel alloys, combinations thereof, and the like) and/or one or more polymers (e.g., silicone, polyvinyl chloride, latex, polyurethane, polyethylene, PTFE, nylon, combinations thereof, and the like) as described herein.

Catheter (416) and intermediate portion (434) may have any suitable dimensions. For example, catheter (416) and intermediate portion (434) may have any suitable length that allows those components to be advanced from a point external to the body to a target location. In some variations, the catheter (416) may have a length in the range of between about 20 cm and about 200 cm. Catheter (416) and intermediate portion (434) may have any suitable diameter, such as, for example, about 5.7 French, about 6.1 French, about 7 French, about 8.3 French, between about 5 French and about 9 French, between about 5 French and about 7 French, between about 6 French and about 9 French, and the like. The intermediate portion (434) may be removably attached to the proximal portion (436). In some variations, the bipolar sensor (438) may be removably attached to the intermediate portion (434).

The bipolar or multipolar sensor (438) may be disposed within a distal portion (432) of the sensor assembly (430). The bipolar sensor (438) may comprise a sensor housing (440) coupled to a first electrode (442) and a second electrode (444). The shape, dimensions, and materials of the bipolar sensor (438) may be the same as those described herein with respect to any of the bipolar sensors disclosed such as bipolar sensors (100, 200, 312, 512).

### III. Methods

Also described here are methods for non-invasively generating an EMG signal corresponding to muscle tissue using the systems and devices described herein. The methods described here may permit EMG data to be gathered from difficult to reach tissue and measure locations such as moist body cavities and organ systems. This may have numerous benefits, such as permitting evaluation and diagnosis of a range of neuromuscular conditions. Conventional EMG techniques may be inadequate for generating EMG data in many body cavities and organ systems. For example, NEMG in body cavities and organ systems has limited clinical use due to a higher risk of damage to tissue. SEMG may be inadequate for evaluating neuromuscular function due to its lower spatial resolution and contamination from one or more electrical, mechanical, and movement artifacts. Furthermore, SEMG systems having adhesive surface electrodes may not be appropriate for organ systems and moist body cavities.

Generally, the methods described here include advancing a sensor probe into a body cavity to contact a tissue surface, and receive signal data without penetrating or piercing the intact tissue surface. The signal data may be processed and used to generate electromyography data. It should be appreciated that any of the systems and devices described herein may be used in the methods described herein.

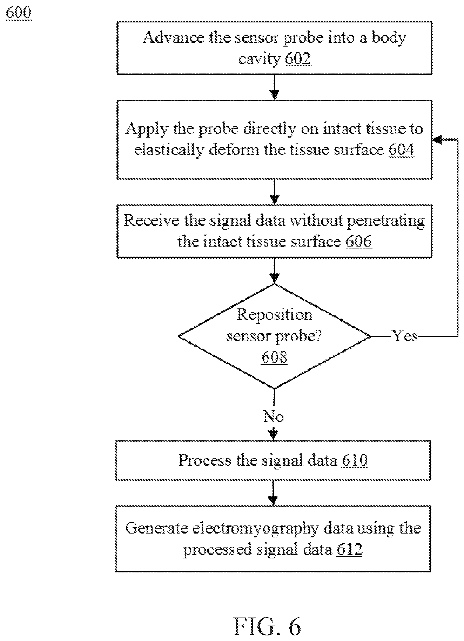

FIG. 6 is a flowchart that generally describes a method of using a sensor probe (600). The process (600) may begin by advancing a sensor probe into a body cavity (602), organ system, or anatomical structure. One or more sensors of the probe may be configured in a bipolar or multipolar configuration. The probe may be rigid or flexible. For example, a rigid probe may be straight, curved, or have one or more straight portions and curved portions. In some variations, the (rigid or flexible) probe may be used in conjunction with a separate delivery device, as described in detail herein.

The probe may be applied directly on intact tissue to elastically deform the tissue surface (604). For example, the probe may atraumatically contact the surface of target tissue and/or a membrane overlying the target tissue. Neither the sensors nor probe penetrates or pierces the intact target tissue. In some variations, the probe may contact the target tissue and/or membrane at an angle between about 60 degrees and about 120 degrees with respect to a surface of the target tissue and/or membrane in contact with the probe. For example, the probe may be perpendicular to the target tissue and/or membrane. In some variations, the probe may contact the target tissue at a midpoint of the muscle to avoid tendinous insertions.

Signal data may be received by the sensors of the probe without penetrating the intact tissue surface (606). For example, the probe may remain in continuous contact with the intact tissue as electrical activity of the target tissue is received by the probe. The tissue surface may be maintained in an unbroken state while applying the one or more sensors of the probe directly on the tissue surface. A determination whether to reposition the sensor probe (608) may be performed. If so, the probe may be moved and steps 604 and 606 repeated. The signal data received by the sensor probe may be processed (610). For example, the electrical activity signal data received by the probe may be amplified by an amplifier of a controller and/or undergo additional filtering in real-time as the data is being received.

Electromyography data may be generated using the processed signal data (612). For example, the processed signal data may be compared against a threshold (e.g., benchmark activity level) to generate EMG data. In some variations, the EMG data may be generated and displayed to a user in real-time to permit the user to determine if the sensor probe should be repositioned. The received and/or processed signal data and generated EMG data may be stored in memory locally, on another device, and/or over a network (e.g., cloud storage, remote server). The sensor probe may be removed from the target tissue and retracted away from the patient.

The systems, devices, and methods described here may be used throughout the body to generate EMG data and permit evaluation of neuromuscular function and/or diagnosis of neuromuscular conditions, examples of which are described in more detail herein.

### A. Transoral EMG example

Conventionally, usage of NEMG in a pharynx and/or tongue is difficult to perform because an awake patient may suffer from one or more of insertion pain, gag reflex, procedure fear, local trauma, and bleeding. Any of these factors may limit needle electrode placement in the transoral cavity. General anesthesia may be used to reduce some of these issues, but the use of anesthesia adds complexity, cost, and other possible complications to the procedure. In some variations, a sensor assembly may comprise a transoral (e.g., airway) probe configured to contact the mucosa and measure electrical activity of one or more of the underlying muscle tissues such as a palatopharyngeus, palatoglossus, musculus uvulae, vocal cord muscles, intrinsic tongue muscle, genioglossus, cricopharyngeus, tensor veli palatini, levator veli palatini, interarytenoideus, cricoarytenoids, cricothyroid, constrictor muscles, upper esophageal sphincter, lower esophageal sphincter, gastroduodenal sphincter, sphincter of Oddi, gastric, and paraspinal, as described in detail herein.

In variations where a rigid sensor probe is used transorally, the rigid sensor probe may be inserted through a patient's mouth and advanced toward one or more muscles to be measured such as in the nasopharynx, oropharynx, hypopharynx, larynx, esophagus, and GI tract. In other variations, a rigid sensor probe may be used with a visualization device such as a rigid straight laryngoscope, rigid laryngeal strobe scope, rigid curved laryngoscope, rigid bronchoscope, rigid esophagoscope, and rigid diverticuloscope to measure.

In variations where a flexible sensor probe is used transorally, the flexible sensor probe may be inserted through a patient's mouth and advanced toward one or more muscles to be measured. In some other variations, a flexible sensor probe may be used with a visualization device such as a flexible fiber optic rhinolaryngoscope, flexible fiber optic bronchoscope, flexible fiber optic esophagoscope. For example, the flexible sensor probe may be slidably advanced through a lumen of the flexible visualization device.

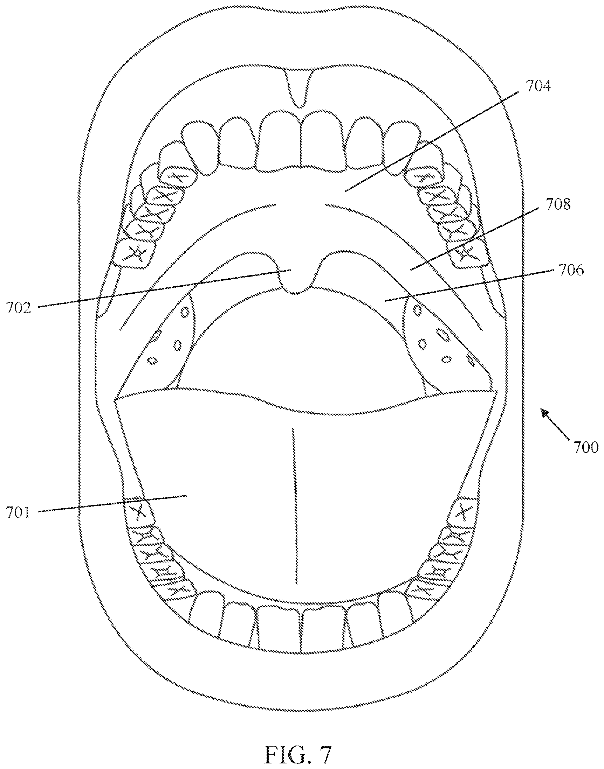

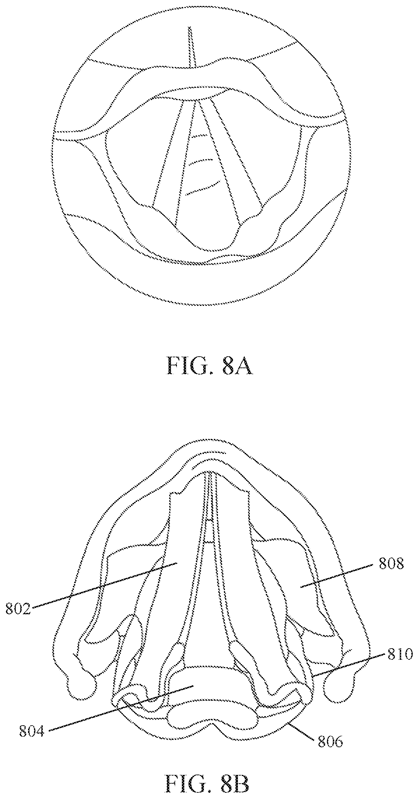

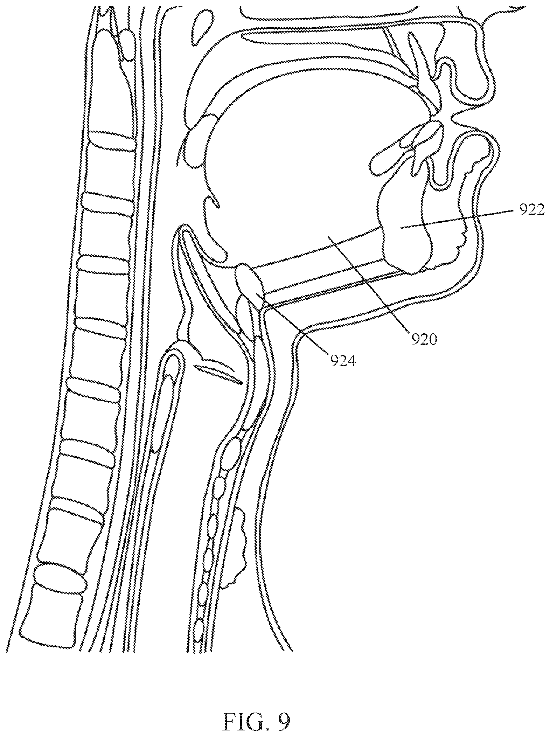

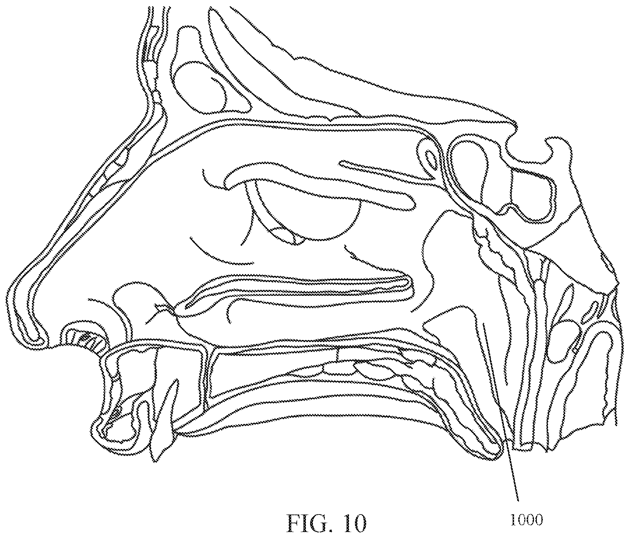

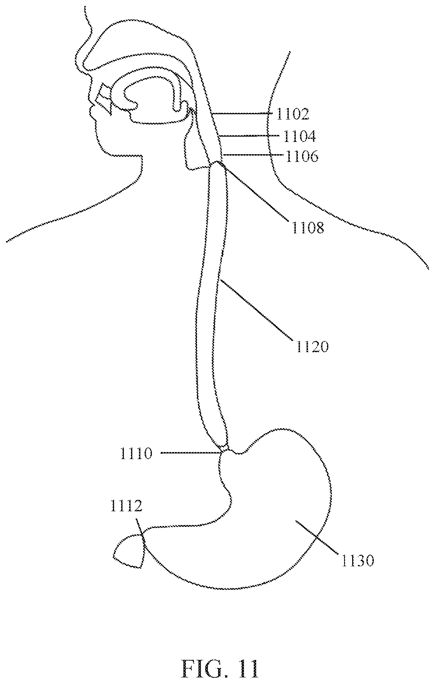

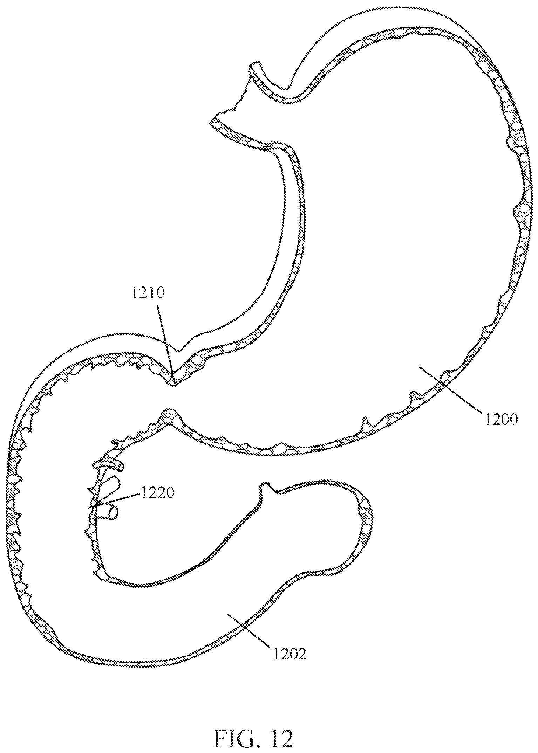

To illustrate some of the methods of using a sensor probe, some of the muscles that may be sensed by the probes described herein are illustrated with respect to the FIGS. 7-12. For example, FIG. 7 is a frontal surface view of the oropharynx (700), FIG. 8A is an axial surface view of the larynx (800), FIG. 8B is an axial view of the larynx musculature, FIG. 9 is a sagittal cross-sectional view of the oropharynx, hypopharynx and larynx, and FIG. 10 is a cross-sectional view of the nasopharynx (1000). FIG. 11 is a cross-sectional view of a portion of the upper gastrointestinal tract and FIG. 12 is a cross-sectional view of the stomach (1200) and duodenum (1202).

For example, the probe may be configured to contact the mucosa overlying a side of the musculus uvulae (702) projecting (e.g., descending) from a posterior edge of the middle of the soft palate (704). The probe may be configured to contact the mucosa overlying one or more of the posterior fasciculus and anterior fasciculus of the palatopharyngeus (706). The probe may be configured to contact the mucosa overlying one or more of the four intrinsic muscles that extend along a length of the tongue including the superior longitudinal muscle that extends along an upper surface of the tongue, the inferior longitudinal muscle that extends along a side of the tongue (701), the vertical muscle located along the middle of the tongue (701) (and which joins the superior and inferior and longitudinal muscles), and the transverse muscle that divides the tongue (701) at the middle. The probe may be configured to contact the mucosa overlying one or more of the genioglossus (920) arising from the mental spine of the mandible (922) and the hyoid (924), and the palatoglossus (708) arising from the palatine aponeurosis of the soft palate (704).

The probe may be configured to contact the mucosa overlying the outer circular layer of the pharynx (902), including one or more of the superior (1102), middle (1104), and inferior (906) constrictor muscles. The superior constrictor muscle (1102) couples to the medial pterygoid plate, the pterygomandibular raphè, and the alveolar process. The middle constrictor muscle (1104) extends from the hyoid. The inferior constrictor muscle (1106) couples to the cricoid and thyroid cartilage. The probe may be configured to contact the mucosa overlying the thyroarytenoid muscle (e.g., vocalis) (802) coupled between the inner surface of the thyroid cartilage and the anterior surface of the arytenoid cartilage.

The probe may be configured to contact the mucosa overlying one or more of the tensor veli palatini muscle and levator veli palatini muscle where the tensor veli palatini is anterior-lateral to the levator veli palatini muscle. The tensor veli palatini muscle is coupled to the medial pterygoid plate of the sphenoid bone and the levator veli palatini muscle is coupled to the temporal bone. The probe may be configured to contact the mucosa overlying the inferior pharyngeal constrictor (e.g., cricopharyngeus) that arises from the sides of the cricoid and thyroid cartilage. The probe may be configured to contact the mucosa overlying the interarytenoideus (804) located in the posterior larynx. The probe may be configured to contact the mucosa overlying the cricoarytenoid muscle (e.g., posterior and lateral cricoarytenoid muscle) (806) located between the cricoid cartilage and arytenoid cartilage. The probe may be configured to contact the mucosa overlying the cricothyroid muscle (808) of the larynx. The cricothyroid muscle (808) is coupled to the anterolateral aspect of the cricoid and the inferior cornu and the lower lamina of the thyroid cartilage. The probe may be configured to contact the mucosa overlying the cricoarytenoid muscles (e.g., posterior and lateral cricoarytenoid muscle) (810) of the larynx that connects the cricoid cartilage and arytenoid cartilage.

### B. Transnasal EMG example

In some variations, a sensor assembly may comprise a transnasal probe configured to contact and measure electrical activity of one or more muscle tissues such as the Eustachian tube (1002) that links the nasopharynx to the middle ear, superior constrictor (1004), and cervical spine (1006). In variations where a rigid sensor probe is used transnasally, the rigid sensor probe may be inserted through a patient's nostril and advanced toward one or more nasopharynx muscles to be measured. In other variations, a rigid sensor probe may be used with a visualization device such as a rigid endoscope to measure one or more nasopharynx muscles.

In variations where a flexible sensor probe is used transnasally, the flexible sensor probe may be inserted through a patient's nostril and advanced toward one or more nasopharynx muscles to be measured. In some other variations, a flexible sensor probe may be used with a visualization device such as a flexible fiber optic rhinolaryngoscope, flexible fiber optic bronchoscope, flexible fiber optic esophagoscope, and flexible gastroduodenoscope to measure one or more tissue surfaces including the nasopharynx, oropharynx, hypopharynx, larynx, esophagus (as described in detail herein), and GI tract. For example, the flexible sensor probe may be slidably advanced through a lumen of the flexible visualization device.

For example, the probe may be configured to contact the mucosa overlying one or more of the upper esophageal sphincter (UES) (e.g., cricopharyngeal part of the inferior pharyngeal constrictor) (1108) that surrounds an upper portion of the esophagus (1120) and lower esophageal sphincter (LES) (e.g., gastroesophageal sphincter) (1110) that surrounds the lower part of the esophagus (1120) at the junction between the esophagus (1120) and the stomach (1130, 1200). The probe may be configured to contact the mucosa overlying one or more of the gastroduodenal sphincter (e.g., pyloric sphincter) (1112, 1210) surrounding the gastroduodenal junction and the sphincter of Oddi (1220) located between the pancreas and duodenum (1202).

### C. Gastrointestinal EMG example

A conventional NEMG needle inserted into an upper and/or lower GI tract may increase the risk of perforation of the viscus and may lead to catastrophic complications related to viscus perforation of the esophagus, duodenum, gallbladder, pancreas, small bowel, large bowel, sigmoid colon, and rectum. In some variations, a sensor assembly may comprise a gastrointestinal probe configured to contact the mucosa overlying muscle tissue such as a lower esophageal sphincter, gastroduodenal juncture, gall bladder, pancreas, small and large intestine sigmoid colon, and rectum. The sensor assembly may comprise one or more flexible and rigid GI endoscopes. The gastrointestinal probe may be disposed in the endoscope using an endoscope port. One or more sensors of the GI probe may be configured to be located on a mucosal surface of the GI tract. In some variations, a distal end of a flexible GI probe may be oriented to be adjacent to a submucosal muscle of interest. For example, the probe may be configured to contact one or more of the lower esophageal sphincter, gastroduodenal sphincter, sphincter of Oddi and gastric tissue, as described in detail herein. In some variations, a rigid probe may slidably advance through a rigid scope such as a rigid esophagoscope, sigmoidoscope, or other operative rigid endoscope.

The EMG data generated from the gastrointestinal probe signal data may be used, in adult and pediatric populations, to evaluate one or more of dysphagia (including the pharyngeal muscles), cricopharyngeus, esophageal muscles, lower esophageal sphincter, pancreatic sphincter, bile duct sphincter, anal sphincter, Ogilvie syndrome, gastrointestinal neoplasm, gallstone ileus, intestinal adhesions causing obstruction, volvulus, intussusception, amyotrophic lateral sclerosis, Brown-Sequard syndrome, central cord syndrome, multiple sclerosis, Parkinson's disease, spina bifida, spinal cord injury, lesions and aging, and diabetes mellitus.

### D. Orbital EMG example

Conventionally, usage of NEMG in and around the eye is difficult to perform. For example, an awake patient may suffer from one or more of insertion pain, procedure fear, local trauma, and bleeding. During the procedure, the needle may need to be repositioned, making patient tolerance and diagnostic compliance even more difficult. A needle electrode further carries a higher risk of intraobrital complications that may lead to double vision, infections, hemorrhage, and/or blindness. General anesthesia may be used to reduce some of these issues, but the use of anesthesia adds complexity, cost, and other possible complications to the procedure. As a result, orbital NEMG is typically limited to specific operating room nerve monitoring conditions.

In some variations, a sensor assembly may comprise an orbital probe configured to contact muscle tissue such as one or more eye muscles such as a medial rectus, lateral rectus, superior oblique, inferior rectus, inferior oblique, and superior rectus. One or more sensors may be configured to be located topically on the eye to measure EMG activity of the extraocular muscles through the conjunctival membrane. In some variations, topical anesthesia may be applied to the conjunctiva and the orbital probe may be subsequently placed directly on the conjunctiva of the overlying muscle. The EMG data generated using the orbital probe signal data may be used to evaluate one or more of Battaglia Neri syndrome, dermatomyositis, diabetic neuropathy, encephalitis, meningitis, multiple sclerosis, myasthenia gravis, Parkinson's disease, subacute sclerosing pan encephalitis, tumor infiltration into muscle, Grave's disease, accommodative esotropia, Brown syndrome, strabismus, convergence insufficiency, dissociated vertical deviation, Duane syndrome, esotropia, exotropia, oculomotor apraxia, abnormal head position, conjugate gaze palsies, internuclear ophthalmoplegia, microvascular cranial nerve palsy, monocular elevation deficiency (double elevator palsy), palsies of the cranial nerves that control eye movement, progressive supranuclear palsy, and pseudo strabismus.

### E. Endovascular EMG example

In some variations, a sensor assembly may comprise an endovascular probe configured to contact muscle tissue such as myocardium, aortic, mitral, pulmonic, tricuspid valves, and all endothelially lined vascular surfaces where adjacent muscle is anatomically known. One or more sensors may be configured to be located on an endothelial surface that anatomically correlates to a muscle such as a myocardium, heart valves, and chordae tendineae to measure EMG activity of those muscles. The EMG data generated using the endovascular probe signal data may be used to evaluate one or more of transient or chronic autonomic dysfunction related to cerebrovascular disease, myocardial infarction or areas of ischemic muscle tissue, valvular disease and muscle function, left ventricular dysfunction arrhythmia, degenerative brain disorders present with myocardial disease, degenerative neurological conditions, cardiomyopathy, neuromuscular disorders, mitochondrial myopathies, dystrophies, cardiac conduction defects, and renovascular disease. Conventional NEMG needle insertion may increase the risk of perforation of the viscus and may lead to catastrophic complications in the myocardium and vascular system.

### F. Orthopedic EMG example

In some variations, a sensor assembly may comprise an orthopedic probe configured to contact muscle directly during surgery in all open muscle operations or via a rigid probe within a synovial cavity of a joint. The sensor assembly may comprise a rigid endoscope placed within the joint during surgical procedures of the joint. One or more sensors may be configured to be located on muscles that traverse through or near a synovial surface such as the shoulder, hip, knee, elbow, ankle, and the like. The EMG data generated from the orthopedic probe signal data may be used, in adult and pediatric populations, to evaluate one or more of muscle activity in open and endoscopic joint surgery, shoulder girdle muscle assessment, hip and knee joint muscle assessment, and any body joint accessible via an open or endoscopic technique.

### G. Urologic EMG example

In some variations, a sensor assembly may comprise a urologic probe configured to contact muscle tissue such as one or more of a urethra, urethral sphincter, and bladder. The sensor assembly may comprise one or more flexible and rigid endoscopes (e.g., cystoscope) advanced using a urethra or suprapubic approach. Conventional NEMG needle insertion may increase the risk of perforation of the viscus and may lead to catastrophic complications in one or more of the urethra and bladder. The EMG data generated from the urologic probe signal data may be used, in adult and pediatric populations, to evaluate one or more of a bladder, spermatic cord, ureteropelvic junction, ureter, ureterovesical junction, prostate, testis, and epididymis conditions. For example, bladder conditions may include overactive bladder, interstitial cystitis, post radiation muscle injury bladder, internal and external urethral sphincter, and bladder neck contracture. Spermatic cord conditions may include pain syndromes associated with cremasteric muscle spasms, varicocele associated with infertility and pain syndromes, and congenital malformations (prenatal in utero-transvaginal). Ureteropelvic junction conditions may include ureteropelvic junction (UPJ) obstruction (e.g., scar, cross-vessel, overactive muscles). Ureter conditions may include mega ureter (e.g., functional, scar, obstruction), ureterectasis (e.g., functional, obstruction), and hydronephrosis (e.g., functional, obstruction). Ureterovesical junction (UVJ) conditions may include vesicoureteral reflux (VUR), overactive bladder (OAB), neurogenic bladder (with or without spinal cord injury), chronic obstructive uropathy, detrusor sphincter dyssynergia, urethral stricture (to check muscle/scar function versus just scar function), and interstitial cystitis. Prostate conditions may include prostatodynia and/or pain syndromes. Testis conditions may include cryptorchidism retractile testis (e.g., overactive cremasteric muscle, true cryptorchid), and verumontanum/ejaculatory duct opening (e.g., obstruction, functional). Epididymis conditions may include epidydimal dilation (e.g., obstruction, functional). Vas Deferens conditions may include vassal immobility syndromes.

### H. Tympanic and transtympanic EMG example

In some variations, a sensor assembly may comprise a tympanic and/or transtympanic probe configured to be contact muscle tissue such as one or more of a stapedius, including round and oval window, cochlea, and labyrinth. The probe may be configured to access the middle ear. In some variations, a probe in contact with the tympanic membrane may permit evaluation of one or more of the tensor tympani, the oval and round window, facial nerve, cochlea, and labyrinth.

In some variations, the probe may be configured to intraoperatively monitor inner ear function for all middle ear, middle fossa, and neurosurgery where monitoring of the vestibular, cochlear, and/or facial nerve may be performed, such as with acoustic neuroma surgery and inner ear surgery. The EMG data generated from the tympanic/transtympanic probe signal data may be used, in adult and pediatric populations, to evaluate one or more of vertigo including vestibular neuronitis, vestibular migraine, Meniere's disease, BPPV, tinnitus, hearing loss from aging, noise, trauma, autoimmune disease, vascular disease, sudden sensorineural hearing loss, ototoxicity, vestibulotoxicity, trauma, and fractures of the temporal bone and skull.

### IV. Examples

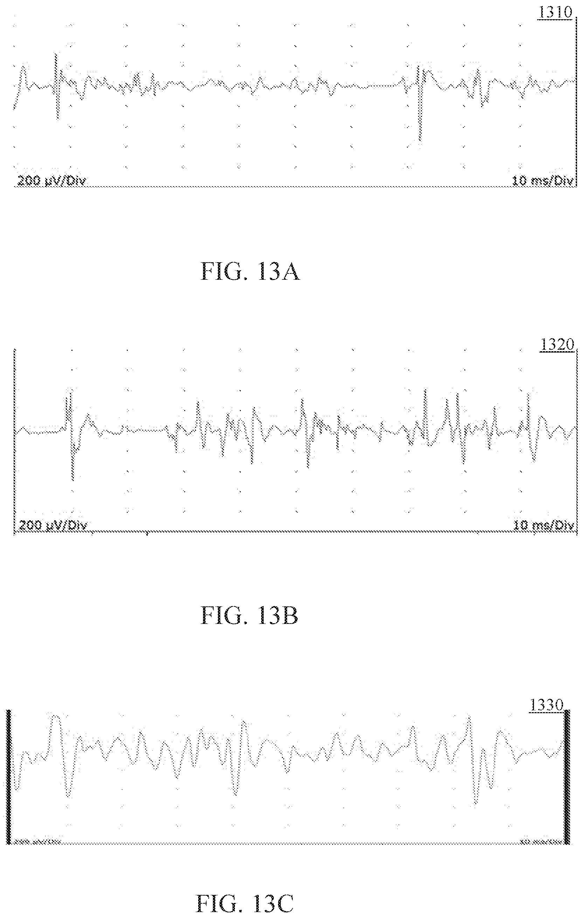

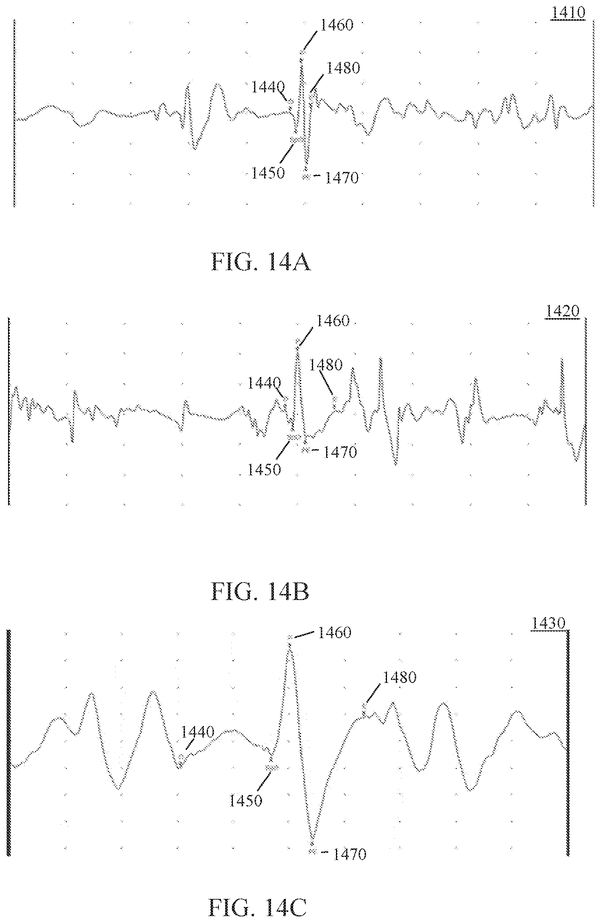

As described herein, EMG data may be generated using the sensor probe assemblies as described herein. For example, FIGS. 13A and 14A are graphs of EMG data (1310, 1410) generated using the systems and methods described herein as applied to a right palatoglossus muscle. FIGS. 13B and 14B are comparative graphs of NEMG data (1320, 1420) of the right palatoglossus muscle of the same patient using a conventional needle electrode. FIGS. 13C and 14C are graphs of SEMG data (1330, 1430) of a right first dorsal interosseous muscle of the same patient using a conventional surface electrode. It should be appreciated that a surface electrode is inadequate to record electrical activity of the palatoglossus muscle due to the moisture (e.g., mucosal lining) between the surface electrode and palatoglossus muscle. Therefore, for the sake of explanation, an external surface SEMG recording of the right first dorsal interosseous muscle (1330, 1430) is provided for comparison with FIGS. 13A-13B and 14A-14B.

As described herein and shown in FIG. 13A and FIG. 13C, the EMG data (1310) generated using the sensor assemblies as described herein has a higher resolution (e.g., detail) than the EMG data (1330) generated using a surface electrode. In contrast, EMG data generated by the sensor assemblies (1310) as described herein and a needle electrode (1320) have similar EMG signal quality due to the close proximity of their sensor electrodes to a motor unit potential of the target tissue. Each of the EMG graphs in FIGS. 13A-13C have a gain and sweep speed set to, respectively, a vertical division of 200 µV/division and a horizontal division of 10 ms/division.

FIGS. 14A-14C correspond to the EMG graphs of respective FIGS. 13A-13C having a shorter timescale such that they depict individual motor unit action potentials (MUAPs). In particular, each of the EMG graphs (1410, 1420, 1430) in FIGS. 14A-14C have a gain and sweep speed set to, respectively, a vertical division of 200 µV/division and a horizontal division of 4 ms/division. A MUAP may have a frequency of between about 30 Hz and about 10 KHz. Each MUAP may comprise a set of characteristics including rise time, amplitude, duration, number of turns, area, phases, thickness, and size index. In particular, each MUAP may comprise a set of points including onset (O - 1440), spike onset (Sp - 1450), peak (P - 1460), peak end (PE - 1470), and end (E - 1480). MUAP characteristics may be calculated from EMG data in a manual, semi-automated, or automated manner. Table 1 lists a set of MUAP characteristics corresponding to FIGS. 13-14.

**Table 1**

| Muscle | Rise (ms) | Amp (µV) | Dur (ms) | Area (mVms) | Phases | Turns | Thickness | Size index |
|---|---|---|---|---|---|---|---|---|
| Palatoglossus (TM-EMG) | 0.34 | 661.77 | 1.39 | 0.18 | 3 | 3 | 0.28 | -0.08 |
| Palatoglossus (Needle) | 0.30 | 561.98 | 3.39 | 0.47 | 3 | 3 | 0.84 | 0.34 |
| Dorsal Interosseous (SEMG) | 1.34 | 1421.36 | 13.17 | 2.64 | 4 | 4 | 1.86 | 2.16 |

Rise time is the duration of a rapid positive-negative deflection (e.g., from spike onset SpO to peak P) and may correspond to a distance of muscle fibers to the electrode. For example, a shorter rise time corresponds to a shorter distance between the muscle fibers and the electrode. Duration may correspond to the time from initial deflection to a final return to baseline (e.g., from onset O to end E). For example, normal MUAPs may have a duration between about 10 ms to about 13 ms, although this range may vary among different muscles. Duration may also correspond to an area of the motor unit. Amplitude may correspond to a maximal peak-to-peak amplitude of a main spike of the MUAP (e.g., from peak P to peak end PE). Amplitude generally corresponds to the action potentials of a set of muscle fibers within the motor unit that are in close proximity to the portion of the electrode in contact with tissue. Phase may correspond to the number of times the action potential crosses the baseline plus one. For example, the MUAP may be monophasic, biphasic, triphasic, or polyphasic. Normal potentials may have three or four phases. A turn may correspond to a potential reversal greater than about 50 µV that does not cross the baseline.

The sensor assemblies as described herein may be configured to elastically deform the tissue surface without penetrating or piercing the intact tissue surface (e.g., intact epithelial surface) while receiving signal data corresponding to EMG data of a motor unit action potential having a rise time of less than about 500 µs. For example, the sensor assemblies as described herein may be configured to receive signal data corresponding to EMG data of a motor unit action potential having a rise time of less than about 300 or 400 µs while maintaining the tissue surface in an intact or unbroken state, e.g. without requiring needle puncture or anatomical dissection to achieve access to the target musculature. In some variations, the sensor probes as described herein may be repositioned when EMG data indicates motor unit action potential rise times above 500 µs. Surface electrodes used for SEMG are not sensitive enough to generate EMG data with rise times of less than about 500 µs due to the large surface area of the surface electrode.

## Claims

1. A sensor assembly (100, 300) for transorally obtaining electromyography, EMG, signals generated from muscles associated with one or more of a patient's soft palate, pharyngeal wall, and tongue, the sensor assembly comprising a first electrode (120, 320), a second electrode (122, 322) electrically isolated from the first electrode (120, 320), and a sensor housing (110, 310) having a length and coupling the first (120, 320) and second (122, 322) electrodes, wherein the first (120, 320) and second (122, 322) electrodes project in parallel from a surface of the sensor housing (110, 310) for a projection length (124) **characterized in that**:
a distance between central longitudinal axes of the first (120, 320) and second electrodes (122, 322) is between 0.30 mm and 2.0 mm, the projection length (124) of the first (120, 320) and second (122, 322) electrodes from the surface of the sensor housing (110, 310) is between 0.5 mm and 3 mm, and a diameter of the first (120, 320) and second (122, 322) electrodes is between 0.3 mm and 0.75 mm, wherein each electrode has a rounded distal end; and
the sensor assembly (100, 300) is configured having:
a distal portion (302) from which the first (120, 320) and second (122, 322) electrodes project for the projection length (124) from the sensor housing (110, 310);
an intermediate portion (304) comprising an elongate body configured for advancement into the patient's mouth for contact with a tissue surface associated with one or more of the soft palate, pharyngeal wall, and tongue; and
a proximal portion (306) configured as a handle (306) for an operator to grasp and control the sensor assembly (100, 300) in use.

2. The sensor assembly (100, 300) of claim 1, wherein the sensor assembly (100, 300) comprises one or more dental markers.

3. The sensor assembly (100, 300) of claim 1, wherein the sensor assembly (100, 300) is configured as a rigid or a flexible probe.

4. The sensor assembly (100, 300) of claim 1, further comprising:
an amplifier (530) coupled to the sensor assembly (100, 300); and
a controller (520) coupled to the sensor assembly (100, 300) and the amplifier (530), the controller (520) comprising a processor (522) and a memory (524), and the controller (520) configured to:
receive signal data corresponding to electrical activity of muscle tissue when the sensor assembly contacts the patient's mouth area associated with the muscle tissue;
amplify the signal data; and
generate electromyography data using the amplified signal data.

5. The sensor assembly (100, 300) of claim 4, wherein the amplifier (530) comprises a pre-amplifier.

6. The sensor assembly (100, 300) of claim 1, comprising one or more ground electrodes.

7. The sensor assembly (100, 300) of claim 1, comprising one or more lead wires (330, 332) respectively coupled to the first (120 320) and second (122, 322) electrodes, the lead wires (330, 332) comprising between 7 strands and 100 strands

8. A method of using a sensor assembly (100, 300) configured for transorally obtaining electromyography, EMG, signals generated from one or more of a patient's soft palate, pharyngeal wall, and tongue areas, the method, comprising:
(i) atraumatically contacting a surface of the patient's soft palate, pharyngeal wall, or tongue with the sensor assembly (100, 300), the sensor assembly (100, 300) comprising a first electrode (120, 320), a second electrode (122, 322), and a sensor housing (110, 310) coupling the first (120, 320) and second (122, 322) electrodes, wherein the first (120, 320) and second (122, 322) electrodes project in parallel from a surface of the sensor housing (110, 310) for a projection length (124),
wherein a distance between central longitudinal axes of the first (120, 320) and second (122, 322) electrodes is between 0.30 mm and 2.0 mm, the projection length (124) of the first (120, 320) and second (122, 322) electrodes from the surface of the sensor housing (110, 310) is between 0.5 mm and 3 mm, and a diameter of the first (120, 320) and second (122, 322) electrodes is between 0.3 mm and 0.75 mm, and wherein each electrode has a rounded distal end; and
the sensor assembly (100, 300) is configured having:
a distal portion (302) from which the first (120, 320) and second (122, 322) electrodes project for the projection length (124) from the sensor housing (110, 310);
an intermediate portion (304) comprising an elongate body configured for advancement into the patient's mouth for contact with a tissue surface associated with one or more of the patient's soft palate, pharyngeal wall, and tongue; and
a proximal portion (306) configured as a handle (306) for an operator to grasp and control the sensor assembly (100, 300) in use; and
(ii) receiving signal data generated from contact of the sensor assembly (100, 300) directly on the tissue surface of one or more of the patient's soft palate, pharyngeal wall, and tongue wherein the rise time for the signal data is less than 500 µs.

9. The method of claim 8, wherein the first (120, 320) and second (122, 322) electrodes are spaced apart by a spacing distance (318), and a first ratio of the spacing distance (318) to the projection length (124) is between 0.075:1 and 1.5:1

10. The method of claim 8, wherein step (i) comprises elastically deforming the tissue surface with the sensor assembly (100, 300), without penetrating or piercing the tissue surface.

11. The sensor assembly of any one of claims 1 to 7, wherein the first and second electrodes are configured to atraumatically contact a tissue surface of associated with one or more muscles in the patient's soft palate, pharyngeal wall, and tongue and receive electrical activity signal data from the portion of the muscle between the electrodes.

12. The method of any one of claims 8 to 10, wherein during steps (i) and (ii), the tissue surface is an overlying membrane of a muscle associated with the patient's palate, pharyngeal wall, or tongue.

## Patentansprüche

1. Sensoranordnung (100, 300) zum transoralen Erhalten von Elektromyographiesignalen, EMG-Signalen, die von Muskeln erzeugt werden, die einem oder mehreren von einem weichen Gaumen, einer Rachenwand und einer Feder eines Patienten zugeordnet sind, die Sensoranordnung umfassend eine erste Elektrode (120, 320), eine zweite Elektrode (122, 322), die von der ersten Elektrode (120, 320) elektrisch isoliert ist, und ein Sensorgehäuse (110, 310), das eine Länge aufweist und die erste (120, 320) und zweite (122, 322) Elektrode koppelt, wobei die erste (120, 320) und zweite (122, 322) Elektrode parallel von einer Oberfläche des Sensorgehäuses (110, 310) um eine Vorsprungslänge (124) vorstehen, **dadurch gekennzeichnet, dass:**
ein Abstand zwischen zentralen Längsachsen der ersten (120, 320) und zweiten Elektroden (122, 322) zwischen 0,30 mm und 2,0 mm liegt, die Vorsprungslänge (124) der ersten (120, 320) und zweiten (122, 322) Elektroden von der Oberfläche des Sensorgehäuses (110, 310) zwischen 0,5 mm und 3 mm liegt und ein Durchmesser der ersten (120, 320) und zweiten (122, 322) Elektroden zwischen 0,3 mm und 0,75 mm liegt, wobei jede Elektrode ein abgerundetes distales Ende aufweist; und
die Sensoranordnung (100, 300) konfiguriert ist, dass sie aufweist:
einen distalen Abschnitt (302), von dem die erste (120, 320) und zweite (122, 322) Elektrode um die Vorsprungslänge (124) aus dem Sensorgehäuse (110, 310) vorstehen;
einen Zwischenabschnitt (304), umfassend einen länglichen Körper, der zum Vorschieben in den Mund des Patienten für den Kontakt mit einer Gewebeoberfläche konfiguriert ist, die einem oder mehreren von dem weichen Gaumen, der Rachenwand und der Zunge zugeordnet ist; und
einen proximalen Abschnitt (306), der als Griff (306) für einen Bediener ausgebildet ist, um die Sensoranordnung (100, 300) im Gebrauch zu greifen und zu steuern.

2. Sensoranordnung (100, 300) nach Anspruch 1, wobei die Sensoranordnung (100, 300) eine oder mehrere Dentalmarkierungen umfasst.

3. Sensoranordnung (100, 300) nach Anspruch 1, wobei die Sensoranordnung (100, 300) als eine starre oder eine flexible Sonde konfiguriert ist.

4. Sensoranordnung (100, 300) nach Anspruch 1, ferner umfassend:
einen Verstärker (530), der an die Sensoranordnung (100, 300) gekoppelt ist; und
eine Steuerung (520), die an die Sensoranordnung (100, 300) und den Verstärker (530) gekoppelt ist, die Steuerung (520) umfassend einen Prozessor (522) und einen Speicher (524), und die Steuerung (520) ist konfiguriert zum:
Empfangen von Signaldaten, die der elektrischen Aktivität von Muskelgewebe entsprechen, wenn die Sensoranordnung den Mundbereich des Patienten kontaktiert, der dem Muskelgewebe zugeordnet ist;
Verstärken der Signaldaten; und
Erzeugen von Elektromyographiedaten unter Verwendung der verstärkten Signaldaten.

5. Sensoranordnung (100, 300) nach Anspruch 4, wobei der Verstärker (530) einen Vorverstärker umfasst.

6. Sensoranordnung (100, 300) nach Anspruch 1, umfassend eine oder mehrere Masseelektroden.

7. Sensoranordnung (100, 300) nach Anspruch 1, umfassend einen oder mehrere Leitungsdrähte (330, 332), die jeweils an die erste (120 320) und zweite (122, 322) Elektrode gekoppelt sind, die Leitungsdrähte (330, 332) umfassend zwischen 7 Litzen und 100 Litzen.

8. Verfahren zur Verwendung einer Sensoranordnung (100, 300), die zum transoralen Erhalten von Elektromyographiesignalen, EMG-Signalen, konfiguriert ist, die von einem oder mehreren von Bereichen des weichen Gaumens, der Rachenwand und Zungenbereichen eines Patienten erzeugt werden, das Verfahren umfassend:
(i) atraumatisches Kontaktieren einer Oberfläche des weichen Gaumens, der Rachenwand oder der Zunge des Patienten mit der Sensoranordnung (100, 300), die Sensoranordnung (100, 300) umfassend eine erste Elektrode (120, 320), eine zweite Elektrode (122, 322) und ein Sensorgehäuse (110, 310), das die erste (120, 320) und zweite (122, 322) Elektrode koppelt, wobei die erste (120, 320) und zweite (122, 322) Elektrode parallel von einer Oberfläche des Sensorgehäuses (110, 310) um eine Vorsprungslänge (124) vorstehen,
wobei ein Abstand zwischen zentralen Längsachsen der ersten (120, 320) und zweiten (122, 322) Elektroden zwischen 0,30 mm und 2,0 mm liegt, die Vorsprungslänge (124) der ersten (120, 320) und zweiten (122, 322) Elektroden von der Oberfläche des Sensorgehäuses (110, 310) zwischen 0,5 mm und 3 mm liegt, und ein Durchmesser der ersten (120, 320) und zweiten (122, 322) Elektroden zwischen 0,3 mm und 0,75 mm liegt und wobei jede Elektrode ein abgerundetes distales Ende aufweist; und
die Sensoranordnung (100, 300) konfiguriert ist, dass sie aufweist:
einen distalen Abschnitt (302), von dem die erste (120, 320) und zweite (122, 322) Elektrode um die Vorsprungslänge (124) aus dem Sensorgehäuse (110, 310) vorstehen;
einen Zwischenabschnitt (304), umfassend einen länglichen Körper, der zum Vorschieben in den Mund des Patienten für den Kontakt mit einer Gewebeoberfläche konfiguriert ist, die einem oder mehreren von dem weichen Gaumen, der Rachenwand und der Zunge des Patienten zugeordnet ist; und
einen proximalen Abschnitt (306), der als ein Griff (306) für einen Bediener ausgebildet ist, um die Sensoranordnung (100, 300) im Gebrauch zu greifen und zu steuern; und
(ii) Empfangen von Signaldaten, die aus dem Kontakt der Sensoranordnung (100, 300) direkt auf der Gewebeoberfläche von einem oder mehreren des weichen Gaumens, der Rachenwand und der Zunge des Patienten erzeugt werden, wobei die Anstiegszeit für die Signaldaten weniger als 500 µs beträgt.

9. Verfahren nach Anspruch 8, wobei die ersten (120, 320) und zweiten (122, 322) Elektroden um einen Beabstandungsabstand (318) beabstandet sind und ein erstes Verhältnis des Beabstandungsabstands (318) zu der Vorsprungslänge (124) zwischen 0,075:1 und 1,5:1 liegt.

10. Verfahren nach Anspruch 8, wobei Schritt (i) das elastische Verformen der Gewebeoberfläche mit der Sensoranordnung (100, 300) umfasst, ohne die Gewebeoberfläche zu durchdringen oder zu durchstechen.

11. Sensoranordnung nach einem der Ansprüche 1 bis 7, wobei die erste und zweite Elektrode konfiguriert sind, um eine Gewebeoberfläche atraumatisch zu kontaktieren, die einem oder mehreren Muskeln im weichen Gaumen, in der Rachenwand und in der Zunge des Patienten zugeordnet ist, und elektrische Aktivitätssignaldaten von dem Abschnitt des Muskels zwischen den Elektroden zu empfangen.

12. Verfahren nach einem der Ansprüche 8 bis 10, wobei während der Schritte (i) und (ii) die Gewebeoberfläche eine darüberliegende Membran eines Muskels ist, der dem Gaumen, der Rachenwand oder der Zunge des Patienten zugeordnet ist.

## Revendications

1. Ensemble capteur (100, 300) permettant d'obtenir par voie transorale des signaux d'électromyographie, EMG, générés par des muscles associés à un ou plusieurs éléments parmi le voile du palais, la paroi pharyngée et la langue d'un patient, l'ensemble capteur comprenant une première électrode (120, 320), une seconde électrode (122, 322) isolée électriquement de la première électrode (120, 320), et un logement de capteur (110, 310) ayant une longueur et accouplant les première (120, 320) et seconde (122, 322) électrodes, dans lequel les première (120, 320) et seconde (122, 322) électrodes font saillie en parallèle à partir d'une surface du logement de capteur (110, 310) sur une longueur de saillie (124) **caractérisé en ce que** :
une distance entre des axes longitudinaux centraux des première (120, 320) et seconde (122, 322) électrodes est comprise entre 0,30 mm et 2,0 mm, la longueur de saillie (124) des première (120, 320) et seconde (122, 322) électrodes à partir de la surface du logement de capteur (110, 310) est comprise entre 0,5 mm et 3 mm, et un diamètre des première (120, 320) et seconde (122, 322) électrodes est compris entre 0,3 mm et 0,75 mm, dans lequel chaque électrode a une extrémité distale arrondie ; et
l'ensemble capteur (100, 300) est configuré comme ayant :
une partie distale (302) à partir de laquelle les première (120, 320) et seconde (122, 322) électrodes font saillie sur la longueur de saillie (124) à partir du logement de capteur (110, 310) ;
une partie intermédiaire (304) comprenant un corps allongé conçu pour être avancé dans la bouche du patient pour un contact avec une surface tissulaire associée à un ou plusieurs éléments parmi le palais mou, la paroi pharyngée et la langue ; et
une partie proximale (306) conçue en tant que poignée (306) pour permettre à un opérateur de saisir et de commander l'ensemble capteur (100, 300) lors de l'utilisation.

2. Ensemble capteur (100, 300) selon la revendication 1, dans lequel l'ensemble capteur (100, 300) comprend un ou plusieurs marqueurs dentaires.

3. Ensemble capteur (100, 300) selon la revendication 1, dans lequel l'ensemble capteur (100, 300) est conçu comme une sonde rigide ou flexible.

4. Ensemble d'entraînement (100, 300) selon la revendication 1, comprenant en outre :
un amplificateur (530) couplé à l'ensemble capteur (100, 300) ; et
un dispositif de commande (520) couplé à l'ensemble capteur (100, 300) et à l'amplificateur (530), le dispositif de commande (520) comprenant un processeur (522) et une mémoire (524), et le dispositif de commande (520) étant configuré pour :
recevoir des données de signal correspondant à une activité électrique de tissu musculaire lorsque l'ensemble capteur entre en contact avec la zone de la bouche du patient associée au tissu musculaire ;
amplifier les données de signal ; et
générer des données d'électromyographie à l'aide des données de signal amplifié.

5. Ensemble capteur (100, 300) selon la revendication 4, dans lequel l'amplificateur (530) comprend un préamplificateur.

6. Ensemble capteur (100, 300) selon la revendication 1, comprenant une ou plusieurs électrodes de masse.

7. Ensemble capteur (100, 300) selon la revendication 1, comprenant un ou plusieurs fils conducteurs (330, 332) couplés respectivement aux première (120 320) et seconde (122, 322) électrodes, les fils conducteurs (330, 332) comprenant entre 7 brins et 100 brins

8. Procédé d'utilisation d'un ensemble capteur (100, 300) configuré pour obtenir par voie transorale des signaux d'électromyographie, EMG, générés à partir d'un ou plusieurs éléments parmi des zones du voile du palais, de la paroi pharyngée et de la langue d'un patient, le procédé comprenant :
(i) la mise en contact de manière atraumatique d'une surface du palais mou, de la paroi pharyngée ou de la langue du patient avec l'ensemble capteur (100, 300), l'ensemble capteur (100, 300) comprenant une première électrode (120, 320), une seconde électrode (122, 322) et un logement de capteur (110, 310) accouplant les première (120, 320) et seconde (122, 322) électrodes, dans lequel les première (120, 320) et seconde (122, 322) électrodes font saillie en parallèle à partir d'une surface du logement de capteur (110, 310) sur une longueur de saillie (124),
dans lequel une distance entre des axes longitudinaux centraux des première (120, 320) et seconde (122, 322) électrodes est comprise entre 0,30 mm et 2,0 mm, la longueur de saillie (124) des première (120, 320) et seconde (122, 322) électrodes à partir de la surface du logement de capteur (110, 310) est comprise entre 0,5 mm et 3 mm, et un diamètre des première (120, 320) et seconde (122, 322) électrodes est compris entre 0,3 mm et 0,75 mm, et dans lequel chaque électrode a une extrémité distale arrondie ; et
l'ensemble capteur (100, 300) est configuré comme ayant :
une partie distale (302) à partir de laquelle les première (120, 320) et seconde (122, 322) électrodes font saillie sur la longueur de saillie (124) à partir du logement de capteur (110, 310) ;
une partie intermédiaire (304) comprenant un corps allongé conçu pour être avancé dans la bouche du patient pour un contact avec une surface tissulaire associée à un ou plusieurs éléments parmi le palais mou, la paroi pharyngée et la langue du patient ; et
une partie proximale (306) conçue en tant que poignée (306) pour permettre à un opérateur de saisir et de commander l'ensemble capteur (100, 300) lors de l'utilisation ; et
(ii) la réception de données de signal générées par contact de l'ensemble capteur (100, 300) directement sur la surface tissulaire d'un ou plusieurs éléments parmi le palais mou, la paroi pharyngée et la langue du patient, dans lequel le temps de montée pour les données de signal est inférieur à 500 µs.

9. Procédé selon la revendication 8, dans lequel les première (120, 320) et seconde (122, 322) électrodes sont espacées d'une distance d'espacement (318), et un premier rapport de la distance d'espacement (318) à la longueur de saillie (124) est compris entre 0,075:1 et 1,5:1

10. Procédé selon la revendication 8, dans lequel l'étape (i) comprend la déformation élastique de la surface tissulaire avec l'ensemble capteur (100, 300), sans pénétrer dans la surface tissulaire, ni la percer.

11. Ensemble capteur selon l'une quelconque des revendications 1 à 7, dans lequel les première et seconde électrodes sont conçues pour entrer en contact de manière atraumatique avec une surface tissulaire associée à un ou plusieurs muscles dans le voile du palais, la paroi pharyngée et la langue du patient et pour recevoir des données de signal d'activité électrique à partir de la partie du muscle entre les électrodes.

12. Procédé selon l'une quelconque des revendications 8 à 10, dans lequel, lors des étapes (i) et (ii), la surface tissulaire est une membrane recouvrant un muscle associé au palais, à la paroi pharyngée ou à la langue du patient.
